# EUROPEAN PATENT APPLICATION

(11) **EP 4 375 300 A1**
(43) Date of publication of application: **29.05.2024**
(21) Application number: 22845466.6
(22) Date of filing: 22.07.2022
(51) Int. Cl.: C07K 16/46, A61P 35/00, A61K 39/395, C07K 16/28

(54) **PHARMACEUTICAL COMPOSITION AND USE**

(30) Priority: 23.07.2021 CN 202110842497
(71) Applicant: Akeso Biopharma, Inc., Zhongshan, Guangdong 528437 (CN); Akeso Pharmaceuticals, Inc., Guangzhou, Guangdong 510799 (CN)
(72) Inventor: WANG, Zhongmin, Zhongshan, Guangdong 528437 (CN); LI, Baiyong, Zhongshan, Guangdong 528437 (CN); XIA, Yu, Zhongshan, Guangdong 528437 (CN)
(74) Representative: Altmann Stößel Dick Patentanwälte PartG mbB
(86) International application number: PCT/CN2022/107522
(87) International publication number: WO 2023/001303

(57) **Abstract**

A pharmaceutical composition comprising an anti-TIGIT antibody or an antigen-binding fragment thereof, and an anti-CTLA4-anti-PD-1 bispecific antibody or an antigen-binding fragment thereof. Specifically, a heavy chain variable region of the antibody contains HCDR1-HCDR3 having amino acid sequences as shown in SEQ ID NOs: 3-5, respectively, and a light chain variable region contains LCDR1-LCDR3 having amino acid sequences as shown in SEQ ID NOs: 8-10, respectively.

## Description

### TECHNICAL FIELD

The present invention relates to the field of pharmaceuticals, particularly to a pharmaceutical composition, which comprises an anti-TIGIT antibody or an antigen-binding fragment thereof, and an anti-CTLA4-anti-PD-1 bispecific antibody or an antigen-binding fragment thereof.

### BACKGROUND

TIGIT (T cell Ig and ITIM domain, also known as WUCAM, Vstm3, or VSIG9) is a member of the poliovirus receptor (PVR)/Nectin family. TIGIT consists of an extracellular immunoglobulin variable region (IgV) domain, a type I transmembrane domain, and an intracellular domain with a classical immunoreceptor tyrosine-based inhibitory motif (ITIM) and an immunoglobulin tail tyrosine (ITT) motif. TIGIT is highly expressed in lymphocytes, especially in effector and regulatory CD4+ T cells, follicular helper CD4+ T cells and effector CD8+ T cells, as well as natural killer (NK) cells (Yu X, Harden K, Gonzalez L C, et al. The surface protein TIGIT suppresses T cell activation by promoting the generation of mature immunoregulatory dendritic cells[J]. Nature immunology, 2009, 10(1): 48).

CD155 (also known as PVR, Necl5 or Tage4), CD112 (also known as PVRL2/nectin 2) and CD113 (also known as PVRL3) are ligands to which TIGIT binds (Martinet L, Smyth M J. Balancing natural killer cell activation through paired receptors[J]. Nature Reviews Immunology, 2015, 15(4): 243-254), and CD155 is a high-affinity ligand for TIGIT. In NK cells, TIGIT binding to ligands CD155 and CD112 can inhibit the killing effect of NK cells on TIGIT high expression cells (Stanietsky N, Simic H, Arapovic J, et al. The interaction of TIGIT with PVR and PVRL2 inhibits human NK cell cytotoxicity[J]. Proceedings of the National Academy of Sciences, 2009, 106(42): 17858-17863). It was reported that the killing effect of CD8+ T cells can be enhanced when PD-1 and TIGIT are blocked simultaneously (Johnston R J, Comps-Agrar L, Hackney J, et al. The immunoreceptor TIGIT regulates antitumor and antiviral CD8+ T cell effector function[J]. Cancer cell, 2014, 26(6): 923-937). In recent studies, TIGIT was found to be an immune checkpoint of NK cells and capable of causing NK cell exhaustion as an inhibitory receptor in the process of tumor progression; it was also demonstrated that anti-TIGIT monoclonal antibodies can reverse NK cell exhaustion and be used as immunotherapy for various tumors (Zhang Q, Bi J, Zheng X, et al., Blockade of the checkpoint receptor TIGIT prevents NK cell exhaustion and elicits potent anti-tumor immunity[J]. Nature immunology, 2018, 19(7): 723-732).

In addition, it was reported that TIGIT blockers alone or in combination with PD-1 blockers plus CD96 blockers could significantly reduce the growth of B16 melanoma in wild-type and CD155-/- mouse models (Li X-Y, Das I, Lepletier A, et al., CD155 loss enhances tumor suppression via combined host and tumor-intrinsic mechanisms. J Clin Invest, 2018, 128:2613-25). CD112R blockers alone or in combination with TIGIT blockers and/or PD-1 blockers could increase cytokine production ability of TILs in ovarian cancer, endometrial cancer and lung tumor (Whelan S, Ophir E, Kotturi MF, et al., PVRIG and PVRL2 Are Induced in Cancer and Inhibit CD8+ T-cell function. Cancer Immunol Res, 2019, 7:257-68).

Anti-TIGIT antibody medicaments, as new immune checkpoint antibody medicaments, have promising utility in a variety of applications, and can be used for immunotherapy of tumors. Tiragolumab developed by Roche is now in phase 3 clinical trials, and it was reported that the combination of the TIGIT monoclonal antibody Tiragolumab and the PD-L1 medicament Tecentriq (atezolizumab) as first-line therapy was well tolerated in patients with PD-L1-positive metastatic non-small cell lung cancer (NSCLC) in phase 2 clinical trials and had a significant effect-a 43% reduction in the risk of disease progression (Exit C. Roche to present first clinical data on novel anti-TIGIT cancer immunotherapy tiragolumab at ASCO[J]). Existing clinical records indicate that TIGIT is an important target for treating non-small cell lung cancer, small cell lung cancer, breast cancer, ovarian cancer, colorectal cancer, melanoma, pancreatic cancer, cervical tumor, multiple myeloma, non-Hodgkin's lymphoma, B-lymphoma, and plasma cell cancer.

The transmembrane receptor PD-1 (programmed cell death protein-1) is a member of the CD28 family, and is expressed in activated T cells, B cells, and myeloid cells. Ligands of PD-1, both PDL1 (programmed cell death 1 ligand 1, or PDL-1) and PDL2 (programmed cell death 1 ligand 2, or PDL-2), are members of the B7 superfamily. PDL1 is expressed in a variety of cells including T cells, B cells, endothelial cells and epithelial cells, and PDL2 is expressed only in antigen presenting cells such as dendritic cells and macrophages.

The PD-1/PDL1 signaling pathway plays an important role in regulating immune tolerance, microbial infection, and tumor immune escape. PD-1 is mainly expressed in immune cells such as T cells, and the ligand PDL1 of PD-1 is highly expressed in a plurality of human tumor tissues. Blocking the PD-1/PDL1 signaling pathway may activate inhibited T cells, which thus attack cancer cells. Blocking the PD-1/PDL1 signaling can promote the proliferation of tumor antigen-specific T cells, activate the tumor cell killing process, and further inhibit local tumor growth (Julie R et al., 2012, N Engl J Med., 366:2455-2465). In addition, tumors with high PDL1 expression are associated with cancers that are difficult to detect (Hamanishi et al., 2007, Proc. Natl. Acad. Sci. USA, 104:3360-5). An effective method is administering an anti-PD-1 antibody to modulate the expression of PD-1. Due to the broad anti-tumor prospects and surprising efficacy of PD-1 antibodies, it is widely accepted in the industry that antibodies targeting the PD-1 pathway will bring about breakthroughs in the treatment of various tumors, e.g., non-small cell lung cancer, renal cell carcinoma, ovarian cancer, and melanoma (Homet M. B., Parisi G., et al., 2015, Semin Oncol., 42(3):466-473), leukemia and anemia (Held SA, Heine A, et al., 2013, Curr Cancer Drug Targets., 13(7):768-74).

Cytotoxic T lymphocyte-associated antigen 4 (CTLA4) and CD28 molecules are very similar in aspects of gene structure, chromosome location, sequence homology and gene expression. Both molecules are receptors of co-stimulatory molecule B7, and mainly expressed on the surface of activated T cells. Binding of CTLA4 to B7 inhibits the activation of mouse and human T cells, and plays a negative regulatory role in T cell activation.

CTLA4 antibodies (or anti-CTLA4 monoclonal antibodies) or CTLA4 ligands can prevent CTLA4 from binding to its natural ligands, thereby blocking the transmission of negative regulatory signals by CTLA4 to T cells and enhancing the reactivity of T cells to various antigens. In this respect, *in-vivo* and *in-vitro* studies are essentially consistent. Currently, there are CTLA4 monoclonal antibodies in clinical trials or approved for treating prostate cancer, bladder cancer, colorectal cancer, gastrointestinal cancer, liver cancer, malignant melanoma, etc. (Grosso JF., Jure-Kunkel MN., 2013, Cancer Immun., 13:5).

Interleukin 2 (IL-2) is produced by T cells. It is a growth factor that regulates T cell subgroups, and an important factor in regulating immune responses. It promotes the proliferation of activated B cells, and participates in antibody responses, hematopoiesis and tumor surveillance. Recombinant human IL-2 has been approved by the U.S. FDA for treating malignant tumors, including melanoma, renal tumors, etc., while a clinical study is currently ongoing for treating chronic viral infections (Chavez, A.R., et al., 2009, Ann. N.Y. Acad. Sci., 1182:p.14-27). CTLA4 and CTLA4 antibodies are important influencing factors of T cell functions and interfere with the immune microenvironment in the body. *In-vitro* and *in-vivo* studies demonstrated that CTLA4 antibodies can specifically relieve the immunosuppression of CTLA4, activate T cells, and induce IL-2 generation, and are promising in wide applications in gene therapy against diseases such as tumors and parasite infections.

In summary, developing a treatment mean or combination administration regimen with lower toxicity and higher efficacy is clinically of great meaning.

### SUMMARY

After intensive studies and creative efforts, the inventors used mammalian cell expression systems to express recombinant human TIGIT as an antigen to immunize mice, and obtained hybridoma cells by fusion of mouse spleen cells and myeloma cells. The inventors obtained a hybridoma cell line LT019 (with an accession number of CCTCC NO: C2020208) by screening a large number of samples.

The inventors surprisingly found that the hybridoma cell line LT019 can secrete a specific monoclonal antibody (designated 26B12) specifically binding to human TIGIT, and the monoclonal antibody can effectively bind to TIGIT, reduce the inhibitory effect of TIGIT on immune cells, promote the activity of T cells, reverse NK cell exhaustion, and enhance the killing effect of immune cells on a tumor. Further, the inventors have creatively prepared humanized anti-human TIGIT antibodies (designated 26B12H1L1, 26B12H4L1, 26B12H2L2, 26B12H3L2, 26B12H2L3, 26B12H3L3, 26B12H1L4, and 26B12H4L4).

The inventors also surprisingly found that antibodies 26B12H1L1, 26B12H4L1, 26B12H2L2, 26B12H3L2, 26B12H2L3, 26B12H3L3, 26B12H1L4, and 26B12H4L4 of the present invention have binding activity to TIGIT and have strong affinity; 26B12H1L1, 26B12H4L1, 26B12H2L2, 26B12H3L2, 26B12H2L3, 26B12H3L3, 26B12H1L4, and 26B12H4L4 can effectively reduce the activity of TIGIT.

The present invention is detailed below.

One aspect of the present invention relates to an anti-TIGIT antibody or an antigen-binding fragment thereof, wherein
the anti-TIGIT antibody comprises HCDR1-HCDR3 contained in a heavy chain variable region set forth in SEQ ID NO: 1 and LCDR1-LCDR3 contained in a light chain variable region set forth in SEQ ID NO: 6;
preferably, according to the IMGT numbering system, the antibody comprises a heavy chain variable region comprising HCDR1-HCDR3 having amino acid sequences set forth in SEQ ID NOs: 3-5, respectively, and a light chain variable region comprising LCDR1-LCDR3 having amino acid sequences set forth in SEQ ID NOs: 8-10, respectively.

In one or more embodiments of the present invention, for the anti-TIGIT antibody or the antigen-binding fragment thereof, an amino acid sequence of the heavy chain variable region of the antibody is selected from SEQ ID NO: 1, SEQ ID NO: 11, SEQ ID NO: 13, SEQ ID NO: 15, and SEQ ID NO: 17; and
an amino acid sequence of the light chain variable region of the antibody is selected from SEQ ID NO: 6, SEQ ID NO: 19, SEQ ID NO: 21, SEQ ID NO: 23, and SEQ ID NO: 25.

In one or more embodiments of the present invention, for the anti-TIGIT antibody or the antigen-binding fragment thereof,
the heavy chain variable region of the antibody has an amino acid sequence set forth in SEQ ID NO: 1, and the light chain variable region of the antibody has an amino acid sequence set forth in SEQ ID NO: 6;
the heavy chain variable region of the antibody has an amino acid sequence set forth in SEQ ID NO: 11, and the light chain variable region of the antibody has an amino acid sequence set forth in SEQ ID NO: 19;
the heavy chain variable region of the antibody has an amino acid sequence set forth in SEQ ID NO: 17, and the light chain variable region of the antibody has an amino acid sequence set forth in SEQ ID NO: 19;
the heavy chain variable region of the antibody has an amino acid sequence set forth in SEQ ID NO: 13, and the light chain variable region of the antibody has an amino acid sequence set forth in SEQ ID NO: 21;
the heavy chain variable region of the antibody has an amino acid sequence set forth in SEQ ID NO: 13, and the light chain variable region of the antibody has an amino acid sequence set forth in SEQ ID NO: 23;
the heavy chain variable region of the antibody has an amino acid sequence set forth in SEQ ID NO: 15, and the light chain variable region of the antibody has an amino acid sequence set forth in SEQ ID NO: 21;
the heavy chain variable region of the antibody has an amino acid sequence set forth in SEQ ID NO: 15, and the light chain variable region of the antibody has an amino acid sequence set forth in SEQ ID NO: 23;
the heavy chain variable region of the antibody has an amino acid sequence set forth in SEQ ID NO: 11, and the light chain variable region of the antibody has an amino acid sequence set forth in SEQ ID NO: 25; or
the heavy chain variable region of the antibody has an amino acid sequence set forth in SEQ ID NO: 17, and the light chain variable region of the antibody has an amino acid sequence set forth in SEQ ID NO: 25.

In one or more embodiments of the present invention, for the anti-TIGIT antibody or the antigen-binding fragment thereof, the antibody comprises a non-CDR region derived from a species other than murine, for example, from a human antibody.

In one or more embodiments of the present invention, for the anti-TIGIT antibody or the antigen-binding fragment thereof, the antibody comprises a heavy chain constant region that is an Ig gamma-1 chain C region (e.g., NCBI ACCESSION: P01857), and a light chain constant region that is an Ig kappa chain C region (e.g., NCBI ACCESSION: P01834).

In one or more embodiments of the present invention, for the anti-TIGIT antibody or the antigen-binding fragment thereof, the anti-TIGIT antibody or the antigen-binding fragment thereof is selected from Fab, Fab', F(ab')₂, Fd, Fv, dAb, a complementarity determining region fragment, a single chain antibody, a humanized antibody, a chimeric antibody, or a diabody.

In one or more embodiments of the present invention, for the anti-TIGIT antibody or the antigen-binding fragment thereof, the antibody binds to TIGIT-mFc with a K_{D} less than 4E-10 or less than 4E-11; preferably, the K_{D} is measured by a Fortebio molecular interaction instrument.

In one or more embodiments of the present invention, for the anti-TIGIT antibody or the antigen-binding fragment thereof, the antibody binds to TIGIT-mFc with an EC₅₀ less than 1.5 nM, less than 1.2 nM, or less than 1 nM; preferably, the EC₅₀ is measured by a flow cytometer.

In some embodiments of the present invention, the anti-TIGIT antibody is a monoclonal antibody.

In some embodiments of the present invention, the anti-TIGIT antibody is a humanized antibody, a chimeric antibody, or a multispecific antibody (e.g., a bispecific antibody).

In some embodiments of the present invention, the antigen-binding fragment is selected from Fab, Fab', F(ab')₂, Fd, Fv, dAb, Fab/c, a complementarity determining region fragment, a single chain antibody (e.g., scFv), a humanized antibody, a chimeric antibody, or a bispecific antibody.

In one or more embodiments of the present invention, for the anti-TIGIT antibody or the antigen-binding fragment thereof, the antibody is an antibody produced by hybridoma cell line LT019 deposited at China Center for Type Culture Collection (CCTCC) with an accession number of CCTCC NO: C2020208.

Another aspect of the present invention relates to an isolated nucleic acid molecule encoding the anti-TIGIT antibody or the antigen-binding fragment thereof according to any aspect of the present invention.

Yet another aspect of the present invention relates to a vector comprising the isolated nucleic acid molecule of the present invention.

Yet another aspect of the present invention relates to a host cell comprising the isolated nucleic acid molecule of the present invention or the vector of the present invention.

Yet another aspect of the present invention relates to a hybridoma cell line LT019 deposited at China Center for Type Culture Collection (CCTCC) with an accession number of CCTCC NO: C2020208.

Yet another aspect of the present invention relates to a conjugate comprising an antibody and a conjugated moiety, wherein the antibody is the anti-TIGIT antibody or the antigen-binding fragment thereof according to any aspect of the present invention, and the conjugated moiety is a detectable label; preferably, the conjugated moiety is a radioisotope, a fluorescent substance, a luminescent substance, a colored substance, or an enzyme.

Yet another aspect of the present invention relates to a kit comprising the anti-TIGIT antibody or the antigen-binding fragment thereof according to any aspect of the present invention or comprising the conjugate of the present invention;
wherein preferably, the kit further comprises a secondary antibody specifically recognizing the antibody; optionally, the secondary antibody further comprises a detectable label, e.g., a radioisotope, a fluorescent substance, a luminescent substance, a colored substance, or an enzyme.

Yet another aspect of the present invention relates to use of the antibody according to any aspect of the present invention or the conjugate of the present invention in preparing a kit for detecting the presence or level of TIGIT in a sample.

Yet another aspect of the present invention relates to a pharmaceutical composition comprising the anti-TIGIT antibody or the antigen-binding fragment thereof according to any aspect of the present invention or the conjugate of the present invention; optionally, the pharmaceutical composition further comprises a pharmaceutically acceptable carrier and/or excipient.

In one or more embodiments of the present invention, the pharmaceutical composition further comprises one or more anti-PD-1 antibodies, or one or more anti-PD-L1 antibodies, e.g., an anti-CTLA4-anti-PD-1 bispecific antibody.

In one or more embodiments of the present invention, for the pharmaceutical composition, the anti-TIGIT antibody or the antigen-binding fragment thereof and the anti-PD-1 antibody or the anti-PD-L1 antibody are present in a mass ratio, on antibody basis, of 1:5-5:1, for example, 1:5, 1:4, 1:3, 1:2, 1:1, 2:1, 3:1, 4:1, or 5:1.

Yet another aspect of the present invention relates to a combination product (e.g., a kit) comprising a first product and a second product in separate packages, wherein the first product comprises the anti-TIGIT antibody or the antigen-binding fragment thereof according to any aspect of the present invention, the conjugate of the present invention, or the pharmaceutical composition according to any aspect of the present invention;
the second product comprises at least one anti-PD-1 antibody or at least one anti-PD-L1 antibody, e.g., an anti-CTLA4-anti-PD-1 bispecific antibody; preferably, the combination product further comprises a third product in a separate package comprising one or more chemotherapeutic drugs;
preferably, the first product and the second product further independently comprise one or more pharmaceutically acceptable adjuvants;
preferably, the combination product further comprises a package insert.

In one or more embodiments of the present invention, for the combination product, the anti-TIGIT antibody or the antigen-binding fragment thereof and the anti-PD-1 antibody or the anti-PD-L1 antibody are present in a mass ratio, on antibody basis, of 1:5-5:1, for example, 1:5, 1:4, 1:3, 1:2, 1:1, 2:1, 3:1, 4:1, or 5:1.

Yet another aspect of the present invention relates to use of the antibody according to any aspect of the present invention, the conjugate of the present invention, the pharmaceutical composition according to any aspect of the present invention or the combination product according to any aspect of the present invention in preparing a medicament for treating and/or preventing a tumor.

The antibody according to any aspect of the present invention, the conjugate of the present invention, the pharmaceutical composition according to any aspect of the present invention or the combination product according to any aspect of the present invention is for use in treating and/or preventing a tumor.

Yet another aspect of the present invention relates to a method for treating and/or preventing a tumor, comprising administering to a subject in need an effective amount of the antibody according to any aspect of the present invention, the conjugate of the present invention, the pharmaceutical composition according to any aspect of the present invention or the combination product according to any aspect of the present invention.

The present invention relates to a method for preventing and/or treating a tumor (particularly a malignant tumor) or an infection or an infectious disease, comprising administering to a subject a therapeutically effective amount of an anti-TIGIT antibody in combination with an anti-CTLA4-anti-PD-1 bispecific antibody, and more preferably further in combination with one or more drugs (e.g., a chemotherapeutic agent or a growth inhibitor, a targeted therapeutic agent, an antibody-drug conjugate, an antimetabolite, an antibiotic, a plant-based anticancer agent and/or a hormonal drug, adriamycin, tamoxifen, megestrol, and/or asparaginase), wherein preferably, the anti-TIGIT antibody, the anti-CTLA4-anti-PD-1 bispecific antibody, and the anti-tumor chemotherapeutic drug are administered simultaneously or sequentially.

In some embodiments, the chemotherapeutic agent or the growth inhibitor is selected from a platinum-based drug (e.g., cisplatin, carboplatin, and oxaliplatin), a fluorouracil antineoplastic drug, cyclophosphamide, pemetrexed, paclitaxel, vinca alkaloids, adriamycin, goserelin, an alkylating agent, an anthracycline, an anti-hormonal agent, an aromatase inhibitor, an anti-androgen agent, a protein kinase inhibitor, a lipid kinase inhibitor, an antisense oligonucleotide, a ribozyme, an antimetabolite, a topoisomerase inhibitor, a cytotoxic agent or an anti-tumor antibiotic, a proteasome inhibitor, an anti-microtubule agent, an EGFR antagonist, a retinoid, a tyrosine kinase inhibitor, a histone deacetylase inhibitor, and a combination thereof.

In some embodiments, the targeted therapeutic agent is selected from a B-raf inhibitor, an MEK inhibitor, a K-ras inhibitor, a c-Met inhibitor, an Alk inhibitor, a phosphatidylin-ositol-3-kinase inhibitor, an Akt inhibitor, an mTOR inhibitor, a dual phosphatidylinositol-3-kinase and mTOR inhibitor, and a combination thereof. In some embodiments, the antibody-drug conjugate comprises a drug selected from the group consisting of: maytansine, monomethyl auristatin E, calicheamicin, esperamicin, and a radioisotope chelating agent.

The present invention relates to a method for preventing and/or treating a tumor (particularly a malignant tumor), comprising administering to a subject a therapeutically effective amount of an anti-TIGIT antibody in combination with an anti-CTLA4-anti-PD-1 bispecific antibody, and more preferably further in combination with one or more chemotherapeutic drugs; preferably, the anti-TIGIT antibody, the anti-CTLA4-anti-PD-1 bispecific antibody, and the chemotherapeutic drug are administered simultaneously or sequentially.

In one or more embodiments of the present invention, the tumor is selected from one or more of the following: pancreatic cancer, breast cancer, ovarian cancer, colorectal cancer, cervical tumor, multiple myeloma, non-Hodgkin's lymphoma, B-lymphoma, plasma cell cancer, head and neck cancer, brain cancer, throat cancer, nasopharyngeal cancer, oesophageal cancer, esophageal squamous cancer, thyroid cancer, mesothelioma, adenocarcinoma (e.g., pancreatic cancer and breast cancer), lung cancer (e.g., non-small cell lung cancer and small cell lung cancer), breast cancer, liver cancer (e.g., hepatocellular carcinoma and hepatobiliary cancer), gastric cancer, gastrointestinal cancer, intestinal cancer (e.g., colon cancer and colorectal cancer), biliary tract cancer (e.g., cholangiocarcinoma), kidney cancer, fallopian tube cancer, endometrial cancer, cervical cancer, bladder cancer, urothelial carcinoma, prostate cancer, testicular cancer, skin cancer, melanoma, myeloma (e.g., multiple myeloma), non-Hodgkin's lymphoma, B-lymphoma, plasma cell carcinoma, leukemia, lymphoma, bone cancer, osteosarcoma, chondrosarcoma, high microsatellite instability (MSI-H) or mismatch repair deficient (dMMR) solid tumors.

In one or more embodiments of the present invention, the anti-CTLA4-anti-PD-1 bispecific antibody comprises:
a first protein functional region targeting PD-1 and
a second protein functional region targeting CTLA4,
wherein the first protein functional region is an immunoglobulin, and the second protein functional region is a single chain antibody, or the first protein functional region is a single chain antibody, and the second protein functional region is an immunoglobulin,
wherein
the immunoglobulin comprises HCDR1-HCDR3 (preferably HCDR1-HCDR3 set forth in SEQ ID NOs: 29-31, respectively) in a heavy chain variable region set forth in SEQ ID NO: 27 and LCDR1-LCDR3 (preferably LCDR1-LCDR3 set forth in SEQ ID NOs: 32-34, respectively) in a light chain variable region set forth in SEQ ID NO: 28; the single chain antibody comprises HCDR1-HCDR3 (preferably HCDR1-HCDR3 set forth in SEQ ID NOs: 37-39, respectively) in a heavy chain variable region set forth in SEQ ID NO: 35 and LCDR1-LCDR3 (preferably LCDR1-LCDR3 set forth in SEQ ID NOs: 40-42, respectively) in a light chain variable region set forth in SEQ ID NO: 36;
   or
the immunoglobulin comprises HCDR1-HCDR3 (preferably HCDR1-HCDR3 set forth in SEQ ID NOs: 37-39, respectively) in a heavy chain variable region set forth in SEQ ID NO: 35 and LCDR1-LCDR3 (preferably LCDR1-LCDR3 set forth in SEQ ID NOs: 40-42, respectively) in a light chain variable region set forth in SEQ ID NO: 36; the single chain antibody comprises HCDR1-HCDR3 (preferably HCDR1-HCDR3 set forth in SEQ ID NOs: 29-31, respectively) in a heavy chain variable region set forth in SEQ ID NO: 27 and LCDR1-LCDR3 (preferably LCDR1-LCDR3 set forth in SEQ ID NOs: 32-34, respectively) in a light chain variable region set forth in SEQ ID NO: 28.

In a specific embodiment, for the anti-CTLA4-anti-PD-1 bispecific antibody according to the present invention,
an amino acid sequence of the heavy chain variable region of the immunoglobulin is selected from SEQ ID NO: 27 and SEQ ID NO: 43, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; an amino acid sequence of the light chain variable region of the immunoglobulin is selected from SEQ ID NO: 28 and SEQ ID NO: 44, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; an amino acid sequence of the heavy chain variable region of the single chain antibody is selected from SEQ ID NO: 35, SEQ ID NO: 45, SEQ ID NO: 46, SEQ ID NO: 47, and SEQ ID NO: 48, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; an amino acid sequence of the light chain variable region of the single chain antibody is selected from SEQ ID NO: 36, SEQ ID NO: 49, SEQ ID NO: 50, SEQ ID NO: 51, and SEQ ID NO: 52, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
   or
an amino acid sequence of the heavy chain variable region of the immunoglobulin is selected from SEQ ID NO: 35, SEQ ID NO: 45, SEQ ID NO: 46, SEQ ID NO: 47, and SEQ ID NO: 48, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; an amino acid sequence of the light chain variable region of the immunoglobulin is selected from SEQ ID NO: 36, SEQ ID NO: 49, SEQ ID NO: 50, SEQ ID NO: 51, and SEQ ID NO: 52, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; an amino acid sequence of the heavy chain variable region of the single chain antibody is selected from SEQ ID NO: 27 and SEQ ID NO: 43, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; an amino acid sequence of the light chain variable region of the single chain antibody is selected from SEQ ID NO: 28 and SEQ ID NO: 44, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto.

In a specific embodiment, for the anti-CTLA4-anti-PD-1 bispecific antibody according to the present invention, the bispecific antibody is selected from any one of the following (1) - (20):
(1) the heavy chain variable region of the immunoglobulin has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 35 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 36 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(2) the heavy chain variable region of the immunoglobulin has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 45 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 49 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(3) the heavy chain variable region of the immunoglobulin has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 46 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 50 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(4) the heavy chain variable region of the immunoglobulin has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 35 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 36 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(5) the heavy chain variable region of the immunoglobulin has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 45 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 49 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(6) the heavy chain variable region of the immunoglobulin has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 46 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 50 or a sequence having at least 60%, 70%, 80%, 81%, 82%, 83%, 84%, 85%, 86%, 87%, 88%, 89%, 90%, 91%, 92%, 93%, 94%, 95%, 96%, 97%, 98% or 99% homology thereto;
(7) the heavy chain variable region of the immunoglobulin has an amino acid sequence set forth in SEQ ID NO: 35 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 36 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(8) the heavy chain variable region of the immunoglobulin has an amino acid sequence set forth in SEQ ID NO: 35 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 36 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(9) the heavy chain variable region of the immunoglobulin has an amino acid sequence set forth in SEQ ID NO: 45 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 49 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(10) the heavy chain variable region of the immunoglobulin has an amino acid sequence set forth in SEQ ID NO: 45 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 49 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(11) the heavy chain variable region of the immunoglobulin has an amino acid sequence set forth in SEQ ID NO: 46 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 50 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(12) the heavy chain variable region of the immunoglobulin has an amino acid sequence set forth in SEQ ID NO: 46 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 50 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(13) the heavy chain variable region of the immunoglobulin has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 47 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 51 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(14) the heavy chain variable region of the immunoglobulin has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 48 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 52 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(15) the heavy chain variable region of the immunoglobulin has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 47 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 51 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(16) the heavy chain variable region of the immunoglobulin has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 48 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 52 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(17) the heavy chain variable region of the immunoglobulin has an amino acid sequence set forth in SEQ ID NO: 47 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 51 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(18) the heavy chain variable region of the immunoglobulin has an amino acid sequence set forth in SEQ ID NO: 48 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 52 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 28 or a sequence having at least 60%, 70%, 80%, 81%, 82%, 83%, 84%, 85%, 86%, 87%, 88%, 89%, 90%, 91%, 92%, 93%, 94%, 95%, 96%, 97%, 98% or 99% homology thereto;
(19) the heavy chain variable region of the immunoglobulin has an amino acid sequence set forth in SEQ ID NO: 47 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 51 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
   and
(20) the heavy chain variable region of the immunoglobulin has an amino acid sequence set forth in SEQ ID NO: 48 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 52 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto.

In a specific embodiment, for the anti-CTLA4-anti-PD-1 bispecific antibody according to the present invention,
the heavy chain of the immunoglobulin has an amino acid sequence set forth in SEQ ID NO: 53 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain thereof has an amino acid sequence set forth in SEQ ID NO: 54 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto.

In a specific embodiment, for the anti-CTLA4-anti-PD-1 bispecific antibody according to the present invention, the first protein functional region is linked to the second protein functional region either directly or via a linker fragment, and/or the heavy chain variable region of the single chain antibody is linked to the light chain variable region of the single chain antibody either directly or via a linker fragment. In a specific embodiment, for the anti-CTLA4-anti-PD-1 bispecific antibody according to the present invention, the linker fragment is (GGGGS)n, n being a positive integer; preferably, n is 1, 2, 3, 4, 5, or 6.

In a specific embodiment, for the anti-CTLA4-anti-PD-1 bispecific antibody according to the present invention, the numbers of the first protein functional region and the second protein functional region are each independently 1, 2, or more.

In a specific embodiment, for the anti-CTLA4-anti-PD-1 bispecific antibody according to the present invention, the single chain antibody (preferably the heavy chain variable region) is linked to the C terminus of the heavy chain of the immunoglobulin.

In a specific embodiment, for the anti-CTLA4-anti-PD-1 bispecific antibody according to the present invention,
the immunoglobulin is of human IgG1 subtype,
wherein, according to the EU numbering system, the heavy chain constant region of the immunoglobulin has one of the combinations of the following mutations:
   L234A and L235A; or
   L234A and G237A; or
   L235A and G237A; or
   L234A, L235A, and G237A.

In a specific embodiment, for the anti-CTLA4-anti-PD-1 bispecific antibody according to the present invention, the bispecific antibody comprises:
a first protein functional region targeting PD-1 and
a second protein functional region targeting CTLA4,
the number of the first protein functional region is 1, and the number of the second protein functional region is 2;
wherein the first protein functional region is an immunoglobulin, and the second protein functional region is a single chain antibody;
the heavy chain of the immunoglobulin has an amino acid sequence set forth in SEQ ID NO: 53 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain thereof has an amino acid sequence set forth in SEQ ID NO: 54 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
the heavy chain variable region of the single chain antibody has an amino acid sequence set forth in SEQ ID NO: 48 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 52 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
the single chain antibody is linked to the C terminus of the heavy chain of the immunoglobulin;
the first protein functional region is linked to the second protein functional region via a first linker fragment; the heavy chain variable region of the single chain antibody is linked to the light chain variable region of the single chain antibody via a second linker fragment; the first linker fragment and the second linker fragment are identical or different;
preferably, the first linker fragment and the second linker fragment each have an amino acid sequence independently selected from SEQ ID NO: 55 and SEQ ID NO: 56;
preferably, amino acid sequences of the first linker fragment and the second linker fragment are set forth in SEQ ID NO: 56.

In a specific embodiment, the heavy chain of the anti-CTLA4-anti-PD-1 bispecific antibody according to the present invention has an amino acid sequence set forth in SEQ ID NO: 57, the light chain has an amino acid sequence set forth in SEQ ID NO: 59, and the bispecific antibody has a structure of IgG-scFv, wherein the IgG part is an anti-PD1 antibody, and the scFv part is an anti-CTLA4 antibody,
wherein the HCDR1 sequence of the anti-PD1 antibody is set forth in SEQ ID NO: 61, the HCDR2 sequence is set forth in SEQ ID NO: 62, the HCDR3 sequence is set forth in SEQ ID NO: 63, the VH sequence is set forth in SEQ ID NO: 73, the LCDR1 sequence of the anti-PD1 antibody is set forth in SEQ ID NO: 70, the LCDR2 sequence is set forth in SEQ ID NO: 71, the LCDR3 sequence is set forth in SEQ ID NO: 72, and the VL sequence is set forth in SEQ ID NO: 76;
the HCDR1 sequence of the anti-CTLA4 antibody is set forth in SEQ ID NO: 64, the HCDR2 sequence is set forth in SEQ ID NO: 65, the HCDR3 sequence is set forth in SEQ ID NO: 66, the VH sequence is set forth in SEQ ID NO: 74, the LCDR1 sequence of the anti-CTLA4 antibody is set forth in SEQ ID NO: 67, the LCDR2 sequence is set forth in SEQ ID NO: 68, the LCDR3 sequence is set forth in SEQ ID NO: 69, and the VL sequence is set forth in SEQ ID NO: 75.

Another aspect of the present invention relates to a unit formulation, preferably used for treating a tumor, and comprising 1-10000 mg (preferably 10-1000 mg, preferably 50-500 mg, 100-400 mg, 150-300 mg, 150-250 mg or 200 mg) of the anti-TIGIT antibody according to any aspect of the present invention and 1-10000 mg (preferably 1-1000 mg, preferably 50-500 mg, 100-400 mg, 150-300 mg, 150-250 mg, 200 mg or 100 mg) of the anti-CTLA4-anti-PD-1 bispecific antibody according to any aspect of the present invention, and optionally one or more of the chemotherapeutic drugs (such as a platinum-based drug and/or a fluorouracil antineoplastic drug) according to the present invention, wherein the anti-TIGIT antibody, the anti-CTLA4-anti-PD-1 bispecific antibody and the chemotherapeutic drug are packaged separately.

The present invention relates to a method for preventing or treating cancer or a tumor, wherein the method comprises administering to a subject in need thereof one or more unit formulations according to the present invention; preferably, the anti-CTLA4-anti-PD-1 bispecific antibody, the anti-TIGIT antibody and the chemotherapeutic drug in the unit formulation are each administered separately. Another aspect of the present invention relates to a single dose unit, preferably used for treating a tumor, and comprising 0.1-10000 mg (preferably 1-1000 mg, preferably 50-500 mg, 100-400 mg, 150-300 mg, 150-250 mg, 200 mg or 100 mg) of the anti-TIGIT antibody according to any aspect of the present invention and 0.1-10000 mg (preferably 1-1000 mg, preferably 50-500 mg, 100-400 mg, 150-300 mg, 150-250 mg, 200 mg or 100 mg) of the anti-CTLA4-anti-PD-1 bispecific antibody according to any aspect of the present invention.

In one or more embodiments of the present invention, the anti-TIGIT antibody, the anti-CTLA4-anti-PD-1 bispecific antibody and/or the chemotherapeutic drug are in a form suitable for intravenous injection or intravenous drip infusion, preferably in a liquid form.

In one or more embodiments of the present invention, the administration of the effective amount of the anti-TIGIT antibody according to any aspect of the present invention and/or the anti-CTLA4-anti-PD-1 bispecific antibody according to any aspect of the present invention to the subject is before or after a surgical treatment and/or before or after a radiation therapy.

In one or more embodiments of the present invention, the unit dose of the anti-TIGIT antibody according to any aspect of the present invention and/or the anti-CTLA4-anti-PD-1 bispecific antibody according to any aspect of the present invention is 0.1-100 mg, preferably 1-10 mg, per kg body weight; alternatively, the unit dose of the anti-TIGIT antibody according to any aspect of the present invention and/or the anti-CTLA4-anti-PD-1 bispecific antibody according to any aspect of the present invention is 10-1000 mg, preferably 50-500 mg, 100-400 mg, 150-300 mg, 150-250 mg, or 200 mg, in each subject;
preferably, the dose is administered from twice daily to about once every other day, or once every 3 days, 4 days, 5 days, 6 days, 10 days, 1 week, 2 weeks, 3 weeks, 4 weeks, 5 weeks, or 6 weeks;
preferably, the route of administration is intravenous drip infusion or intravenous injection.

The variable regions of the light chain and the heavy chain determine the binding of the antigen; the variable region of each chain contains three hypervariable regions called complementarity determining regions (CDRs); CDRs of the heavy chain (H) comprise HCDR1, HCDR2 and HCDR3, and CDRs of the light chain (L) comprise LCDR1, LCDR2 and LCDR3, which are named by Kabat et al., see Bethesda M.d., Sequences of Proteins of Immunological Interest, Fifth Edition, NIH Publication 1991; 1-3:91-3242. Given the known sequences of the heavy and light chain variable regions of an antibody, there are several methods for determining the CDR regions of the antibody, including the Kabat, IMGT, Chothia and AbM numbering systems. However, the application of all the definitions of CDRs for an antibody or its variant shall fall within the scope of the terms defined and used herein. If an amino acid sequence of the variable region of the antibody is known, those skilled in the art can generally determine a particular CDR, without relying on any experimental data beyond the sequence itself.

Preferably, CDRs may also be defined by the IMGT numbering system, see Ehrenmann, Francois, Quentin Kaas, and Marie-Paule Lefranc. IMGT/3Dstructure-DB and IMGT/DomainGapAlign: a database and a tool for immunoglobulins or antibodies, T cell receptors, MHC, IgSF and MhcSF. Nucleic acids research 2009; 38(suppl_1): D301-D307.

The amino acid sequences of the CDRs of the monoclonal antibody sequences are analyzed according to the IMGT definition by technical means well known to those skilled in the art, for example by using the VBASE2 database.

The antibodies 26B12, 26B12H1L1, 26B12H4L1, 26B12H2L2, 26B12H3L2, 26B12H2L3, 26B12H3L3, 26B12H1L4, and 26B12H4L4 involved in the present invention have the same CDRs:
the 3 CDRs of their heavy chain variable regions have the following amino acid sequences:
HCDR1: GHSFTSDYA (SEQ ID NO: 3)
HCDR2: ISYSDST (SEQ ID NO: 4)
HCDR3: ARLDYGNYGGAMDY (SEQ ID NO: 5);
the 3 CDRs of their light chain variable regions have the following amino acid sequences:
   LCDR1: QHVSTA (SEQ ID NO: 8)
   LCDR2: SAS (SEQ ID NO: 9)
   LCDR3: QQHYITPWT (SEQ ID NO: 10).

In the present invention, unless otherwise defined, the scientific and technical terms used herein have the meanings generally understood by those skilled in the art. In addition, the laboratory operations of cell culture, molecular genetics, nucleic acid chemistry and immunology used herein are the routine procedures widely used in the corresponding fields. Meanwhile, in order to better understand the present invention, the definitions and explanations of the relevant terms are provided below.

As used herein, when referring to the amino acid sequence of TIGIT (NCBI GenBank ID: NP_776160.2), it includes the full length of TIGIT protein, or an extracellular immunoglobulin variable region (IgV) domain or a fragment comprising an extracellular immunoglobulin variable region (IgV) domain; also included are fusion proteins of TIGIT, e.g., a fragment fused to an Fc protein fragment of a mouse or human IgG (mFc or hFc). However, those skilled in the art will appreciate that in the amino acid sequence of the TIGIT protein, mutations or variations (including but not limited to, substitutions, deletions and/or additions) may naturally occur or can be artificially introduced without affecting biological functions thereof. Therefore, in the present invention, the term "TIGIT protein" or "TIGIT" shall include all such sequences, including the sequences set forth and natural or artificial variants thereof. Moreover, when describing the sequence fragment of the TIGIT protein, it includes not only the sequence fragment but also a corresponding sequence fragment in natural or artificial variants thereof.

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). Antibody light chains are classified into κ and λ light chains. Heavy chains are classified into µ, δ, γ, α, or ε. Isotypes of antibodies are defined as IgM, IgD, IgG, IgA, and IgE. In light chains and heavy chains, the variable region and constant region are linked by a "J" region of about 12 or more amino acids, and the heavy chain further comprises a "D" region of about 3 or more amino acids. Each heavy chain consists of a heavy chain variable region (V_{H}) and a heavy chain constant region (C_{H}). The heavy chain constant region consists of 3 domains (C_{H1}, C_{H2}, and C_{H3}). Each light chain consists of a light chain variable region (V_{L}) and a light chain constant region (C_{L}). The light chain constant region consists of one domain C_{L}. The constant region of the antibody can mediate the binding of immunoglobulins to host tissues or factors, including the binding of various cells of the immune system (e.g., effector cells) to the first component (C1q) of the classical complement system. The V_{H} and V_{L} regions can be further subdivided into hypervariable regions (called complementarity determining regions, or CDRs), and conservative regions called framework regions (FRs) are distributed between the CDRs. Each V_{H} and V_{L} consists of 3 CDRs and 4 FRs arranged from the amino terminus to the carboxyl terminus in the following order: FR1, CDR1, FR2, CDR2, FR3, CDR3, FR4. The variable regions (V_{H} and V_{L}) of each heavy chain/light chain pair form antigen-binding sites, respectively. The assignment of amino acids to the regions or domains is based on Kabat Sequences of Proteins of Immunological Interest (National Institutes of Health, Bethesda, M.d. (1987 and 1991)), or Chothia & Lesk J. Mol. Biol. 1987; 196:901-917; Chothia et al. Nature 1989; 342:878-883 or the definition of the IMGT numbering system, see the definition in Ehrenmann, Francois, Quentin Kaas, and Marie-Paule Lefranc. "IMGT/3Dstructure-DB and IMGT/DomainGapAlign: a database and a tool for immunoglobulins or antibodies, T cell receptors, MHC, IgSF and MhcSF." Nucleic acids research 2009; 38(suppl_1): D301-D307. The term "antibody" is not limited by any specific method for producing the antibody. For example, the antibody includes, in particular, a recombinant antibody, a monoclonal antibody and a polyclonal antibody. The antibody can be antibodies of different isotypes, e.g., IgG (e.g., subtypes IgG1, IgG2, IgG3 or IgG4), IgA1, IgA2, IgD, IgE or IgM.

As used herein, the term "antigen-binding fragment", also known as the "antigen-binding portion", refers to a polypeptide comprising the fragment of a full-length antibody, which maintains the ability to specifically bind to the same antigen to which the full-length antibody binds, and/or competes with the full-length antibody for the specific binding to the antigen. See generally, Fundamental Immunology, Ch. 7 (Paul, W., ed., 2nd edition, Raven Press, N.Y. (1989), which is incorporated herein by reference in its entirety for all purposes. The antigen-binding fragment of the antibody can be produced by recombinant DNA technology or by enzymatic or chemical cleavage of an intact antibody. In some cases, the antigen-binding fragment includes Fab, Fab', F(ab')₂, Fd, Fv, dAb, a complementarity determining region (CDR) fragment, a single chain antibody (e.g., scFv), a chimeric antibody, a diabody, and polypeptides comprising at least a portion of the antibody sufficient to impart specific antigen binding ability to them. As used herein, the term "Fd fragment" refers to an antibody fragment consisting of V_{H} and C_{H}1 domains; the term "Fv fragment" refers to an antibody fragment consisting of the V_{L} and V_{H} domains of a single arm of an antibody; the term "dAb fragment" refers to an antibody fragment consisting of a V_{H} domain (Ward et al., Nature 341:544-546 (1989)); the term "Fab fragment" refers to an antibody fragment consisting of V_{L}, V_{H}, C_{L}, and C_{H}1 domains; the term "F(ab')₂ fragment" refers to an antibody fragment comprising two Fab fragments linked by the disulfide bridge on a hinge region.

In some cases, the antigen-binding fragment of the antibody is a single chain antibody (e.g., scFv) in which the V_{L} and V_{H} domains are paired to form a monovalent molecule via a linker that enables them to produce a single polypeptide chain (see, e.g., Bird et al., Science 242:423-426 (1988) and Huston et al., Proc. Natl. Acad. Sci. USA 85:5879-5883 (1988)). Such scFv molecules may have a general structure: NH₂-V_{L}-linker-V_{H}-COOH or NH₂-V_{H}-linker-V_{L}-COOH. An appropriate linker in the prior art consists of GGGGS amino acid sequence repeats or a variant thereof. For example, a linker having the amino acid sequence (GGGGS)₄ may be used, but variants thereof may also be used (Holliger et al., (1993), Proc. Natl. Acad. Sci. USA, 90: 6444-6448). Other linkers that may be used in the present invention are described by Alfthan et al., (1995), Protein Eng., 8:725-731; Choi et al., (2001), Eur. J. Immunol., 31: 94-106; Hu et al., (1996), Cancer Res., 56:3055-3061; Kipriyanov et al., (1999), J. Mol. Biol., 293:41-56; and Roovers et al., (2001), Cancer Immunol.

In some cases, the antigen-binding fragment of the antibody is a diabody, that is, a bivalent antibody, in which the V_{H} and V_{L} domains are expressed on a single polypeptide chain. However, the linker used is too short to allow the pairing of the two domains on one chain. Thus the domains are forced to pair with the complementary domains on another chain and two antigen-binding sites are generated (see, e.g., Holliger P. et al., Proc. Natl. Acad. Sci. USA 90:6444-6448 (1993), and Poljak R. J. et al., Structure 2:1121-1123 (1994)).

In other cases, the antigen-binding fragment of the antibody is a "bispecific antibody", which refers to a conjugate formed from a first antibody (fragment) and a second antibody (fragment) or antibody analog via a linker; the methods of conjugation include, but are not limited to, chemical reaction, gene fusion, and enzymatic reaction. The antigen-binding fragment of the antibody may be a "multispecific antibody" including, for example, a trispecific antibody and a tetraspecific antibody, the former being an antibody with three different kinds of antigen-binding specificity, and the latter being an antibody with four different kinds of antigen-binding specificity. For example, a designed ankyrin repeat protein (DARPin) is linked to an IgG antibody, a scFv-Fc antibody fragment or combinations thereof, such as CN104341529A. An anti-IL-17a fynomer binds to an anti-IL-6R antibody, such as WO2015141862A1.

Antigen-binding fragments of antibodies (e.g., the antibody fragments described above) can be obtained from a given antibody (e.g., monoclonal antibody 26B12H1L1, 26B12H4L1, 26B12H2L2, 26B12H3L2, 26B12H2L3, 26B12H3L3, 26B12H1L4, or 26B12H4L4 provided in the present invention) using conventional techniques known to those skilled in the art (e.g., recombinant DNA technology or enzymatic or chemical cleavage), and the antigen-binding fragments of antibodies are screened for specificity in the same manner as for intact antibodies.

As used herein, the terms "mAb" and "monoclonal antibody" refer to an antibody or a fragment of an antibody that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using hybridoma technology first reported by Kohler et al. (Köhler G, Milstein C. Continuous cultures of fused cells secreting antibody of predefined specificity [J]. Nature, 1975; 256(5517): 495), but can also be obtained using recombinant DNA technology (see, e.g., U.S. Patent 4,816,567).

As used herein, the term "humanized antibody" refers to an antibody or antibody fragment obtained when all or a part of CDRs of a human immunoglobulin (receptor antibody) is replaced by the CDRs of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature 1986; 321:522-525; Reichmann et al., Nature 1988; 332:323-329; Presta, Curr. Op. Struct. Biol., 1992; 2:593-596; and Clark M. Antibody humanization: a case of the 'Emperor's new clothes'? [J]. Immunol. Today, 2000; 21(8): 397-402.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements and reporter genes. In addition, the vector may further comprise a replication initiation site.

As used herein, the term "host cell" refers to cells to which vectors can be introduced, including, but not limited to, prokaryotic cells such as *E. coli* or *bacillus subtilis*, fungal cells such as yeast cells or *aspergillus*, insect cells such as S2 drosophila cells or Sf9, or animal cells such as fibroblasts, CHO cells, COS cells, NSO cells, HeLa cells, GS cells, BHK cells, HEK 293 cells, or human cells. As used herein, the term "specific binding" refers to a non-random binding reaction between two molecules, such as a reaction between an antibody and an antigen it targets. In some embodiments, an antibody specifically binding to an antigen (or an antibody specific to an antigen) means that the antibody binds to the antigen with an affinity (K_{D}) of less than about 10⁻⁵ M, e.g., less than about 10⁻⁶ M, 10⁻⁷ M, 10⁻⁸ M, 10⁻⁹ M or 10⁻¹⁰ M or less.

As used herein, the term "K_{D}" refers to a dissociation equilibrium constant for a specific antibody-antigen interaction, which is used to describe the binding affinity between the antibody and the antigen. A smaller dissociation equilibrium constant indicates a stronger antibody-antigen binding and a higher affinity between the antibody and the antigen. Generally, the antibodies bind to antigens (e.g., TIGIT protein) with a dissociation equilibrium constant (K_{D}) of less than about 10⁻⁵ M, e.g., less than about 10⁻⁶ M, 10⁻⁷ M, 10⁻⁸ M, 10⁻⁹ M or 10⁻¹⁰ M or less. K_{D} can be determined using methods known to those skilled in the art, e.g., using a Fortebio molecular interaction instrument.

As used herein, the terms "monoclonal antibody" and "mAb" have the same meaning and are used interchangeably; the terms "polyclonal antibody" and "pAb" have the same meaning and are used interchangeably; the terms "polypeptide" and "protein" have the same meaning and are used interchangeably. Besides, as used herein, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient. Such carriers and/or excipients are well known in the art (see, e.g., *Remington's Pharmaceutical Sciences*, edited by Gennaro AR, 19^{th} Ed., Pennsylvania, Mack Publishing Company, 1995), including but not limited to: pH regulators, surfactants, adjuvants and ionic strength enhancers. For example, the pH regulators include, but are not limited to, phosphate buffer; the surfactants include, but are not limited to, cationic, anionic or non-ionic surfactants, such as Tween-80; the ionic strength enhancers include, but are not limited to, sodium chloride.

As used herein, the term "effective amount" refers to an amount sufficient to obtain or at least partially obtain a desired effect. For example, a prophylactically effective amount against a disease (e.g., a tumor) refers to an amount sufficient to prevent, stop, or delay the onset of the disease (e.g., a tumor); a therapeutically effective amount refers to an amount sufficient to cure or at least partially stop diseases and complications thereof in patients suffering from the disease.

As used herein, the terms "hybridoma" and "hybridoma cell line" are used interchangeably, and when referring to the terms "hybridoma" and "hybridoma cell line", they also include subclones and progeny cells of the hybridoma.

The term "single dose unit" means a single pharmaceutical dosage form, such as an injection, e.g. placed in an ampoule, comprising the anti-CTLA4-anti-PD-1 bispecific antibody, or the anti-TIGIT antibody according to the present invention to be administered to a subject at time points of a regimen, preferably per kg body weight of the subject. In a specific embodiment of the present invention, the regimen comprises, for example, administration of a single dose unit according to an administration cycle of from twice daily to about once every other day, or once every 3 days, 4 days, 5 days, 6 days, 10 days, 1 week, 2 weeks, 3 weeks, 4 weeks, 5 weeks, or 6 weeks.

In the present invention, the terms "first" (e.g., first protein functional region or first linker fragment) and "second" (e.g., second protein functional region or second linker fragment) are used for distinguishing or clarity in expression and do not carry typical sequential meanings, unless otherwise specified.

A "therapeutically effective amount" or "therapeutically effective dose" of a drug or therapeutic agent is any amount of a drug that, when used alone or in combination with another therapeutic agent, protects a subject from the onset of a disease or promotes disease regression as evidenced by reduction in the severity of disease symptoms, increase in the frequency and duration of disease symptom-free periods, or the prevention of damage or disability caused by the affliction of the disease. The ability of a therapeutic agent to promote disease regression can be evaluated using a variety of methods known to skilled practitioners, such as in a human subject in clinical trials, in an animal model system that predicts the efficacy in humans, or by determining the activity of the drug in an *in vitro* assay.

The "prophylactically effective amount" of a drug refers to any amount of a drug that inhibits the occurrence or recurrence of cancer when administered, alone or in combination with an antineoplastic agent, to a subject at risk of developing cancer (e.g., a subject having a premalignant condition) or a subject at risk of recurrence of cancer. In some embodiments, the prophylactically effective amount completely prevents the occurrence or recurrence of cancer. "Inhibiting" the occurrence or recurrence of cancer means reducing the possibility of the occurrence or recurrence of cancer or completely preventing the occurrence or recurrence of cancer.

### Beneficial effects of the present invention

The monoclonal antibodies of the present invention can specifically bind to TIGIT very well and have strong affinity. They reduce the inhibitory effect of TIGIT on immune cells, promote the activity of T cells, reverse NK cell exhaustion, and enhance the killing effect of immune cells on tumors. The anti-TIGIT antibody can be used for effectively treating or preventing tumors alone or in combination with the anti-CTLA4-anti-PD-1 bispecific antibody (and/or a chemotherapeutic drug). The anti-TIGIT antibody has a pharmacological effect of effectively inhibiting tumor growth in combination with the anti-CTLA4-anti-PD-1 bispecific antibody, and is superior to the anti-TIGIT antibody alone or the single anti-CTLA4-anti-PD-1 bispecific antibody alone, thus having good application prospects and market value.

### BRIEF DESCRIPTION OF THE DRAWINGS

FIG. 1: The results of assays for the activity of 26B12H1L1, 26B12H2L2, 26B12H2L3, 26B12H3L2 and RG6058 antibodies binding to TIGIT-mFc.
FIG. 2: The results of assays for the activity of 26B12H3L3, 26B12H1L4, 26B12H4L1, 26B12H4L4 and RG6058 antibodies binding to TIGIT-mFc.
FIG. 3: The results of assays for the activity of 26B12H1L1, 26B12H2L2, 26B12H2L3, 26B12H3L2 and RG6058 antibodies competing with human CD155-hFc-Biotin for binding to TIGIT-mFc.
FIG. 4: The results of assays for the activity of 26B12H3L3, 26B12H1L4, 26B12H4L1, 26B12H4L4 and RG6058 antibodies competing with human CD155-hFc-Biotin for binding to TIGIT-mFc.
FIG. 5: The results of assays for the affinity constant of 26B12 H3L3 for TIGIT-mFc. The antibody concentrations for the curve pairs from top to bottom are 5 nM, 1.67 nM, 0.557 nM, 0.185 nM and 0.06 nM, respectively.
FIG. 6: The results of assays for the affinity constant of 26B12 H1L1 for TIGIT-mFc. The antibody concentrations for the curve pairs from top to bottom are 5 nM, 1.67 nM, 0.557 nM, 0.185 nM and 0.06 nM, respectively.
FIG. 7: The results of assays for the affinity constant of 26B12 H2L2 for TIGIT-mFc. The antibody concentrations for the curve pairs from top to bottom are 5 nM, 1.67 nM, 0.557 nM, 0.185 nM and 0.06 nM, respectively.
FIG. 8: The results of assays for the affinity constant of 26B12 H2L3 for TIGIT-mFc. The antibody concentrations for the curve pairs from top to bottom are 5 nM, 1.67 nM, 0.557 nM, 0.185 nM and 0.06 nM, respectively.
FIG. 9: The results of assays for the affinity constant of 26B12 H3L2 for TIGIT-mFc. The antibody concentrations for the curve pairs from top to bottom are 5 nM, 1.67 nM, 0.557 nM, 0.185 nM and 0.06 nM, respectively.
FIG. 10: The results of assays for the affinity constant of 26B12 H4L4 for TIGIT-mFc. The antibody concentrations for the curve pairs from top to bottom are 5 nM, 1.67 nM, 0.557 nM, 0.185 nM and 0.06 nM, respectively.
FIG. 11: The results of assays for the affinity constant of 26B12 H1L4 for TIGIT-mFc. The antibody concentrations for the curve pairs from top to bottom are 5 nM, 1.67 nM, 0.557 nM, 0.185 nM and 0.06 nM, respectively.
FIG. 12: The results of assays for the affinity constant of 26B12 H4L1 for TIGIT-mFc. The antibody concentrations for the curve pairs from top to bottom are 5 nM, 1.67 nM, 0.557 nM, 0.185 nM and 0.06 nM, respectively.
FIG. 13: The results of assays for the affinity constant of RG6058 for TIGIT-mFc. The antibody concentrations for the curve pairs from top to bottom are 5 nM, 1.67 nM, 0.557 nM, 0.185 nM and 0.06 nM, respectively.
FIG. 14: FACS assays for the activity of humanized antibodies 26B12H2L2 and RG6058 binding to 293T-TIGIT cell membrane surface antigen TIGIT.
FIG. 15: FACS assays for the activity of humanized antibodies 26B12H2L2 and RG6058 competing with CD155 for binding to 293T-TIGIT cell membrane surface TIGIT.
FIG. 16: FACS assays for the activity of humanized antibodies 26B12H2L2 and RG6058 competing with CD112 for binding to 293T-TIGIT cell membrane surface TIGIT.
FIG. 17: Assays for the amount of secreted IL-2 after adding the TIGIT antibody into Jurkat-TIGIT and HT1080-aCD3scFv cell systems.
FIG. 18: The efficacy of a hTIGIT-BALB/c transgenic mouse CT26 tumor model.
FIG. 19: The changes in body weight of a hTIGIT-BALB/c transgenic mice CT26 tumor model.
FIG. 20: The efficacy of 26B12H2L2 in combination with the anti-CTLA4-anti-PD-1 bispecific antibody CP004(hG1TM) on a BALB/c-hPD1/hTIGIT transgenic mouse CT26 tumor model.
FIG. 21: The changes in body weight of a BALB/c-hPD1/hTIGIT transgenic mouse CT26 tumor model receiving 26B12H2L2 in combination with the anti-CTLA4-anti-PD-1 bispecific antibody CP004(hG1TM).

### Deposited biological material:

Hybridoma cell line LT019 (TIGIT-26B12) was deposited at China Center for Type Culture Collection (CCTCC) on Oct. 22, 2020, with an accession number of CCTCC NO: C2020208, the depository address being Wuhan University, Wuhan, China, postal code: 430072.

### DETAILED DESCRIPTION

The embodiments of the present invention will be described in detail below with reference to the examples. Those skilled in the art will appreciate that the following examples are only for illustrating the present invention, and should not be construed as limitations to the scope of the present invention. Examples where the specific technologies or conditions are not specified are performed according to the technologies or conditions described in the publications of the art (e.g., see, *Molecular Cloning: A Laboratory Manual*, authored by J. Sambrook et al., and translated by Huang Peitang et al., third edition, Science Press) or according to the package insert. Reagents or instruments used are commercially available conventional products if the manufacturers thereof are not specified. For example, 293T can be purchased from ATCC.

In the following examples of the present invention, BALB/c mice used were purchased from Guangdong Medical Laboratory Animal Center.

In the following examples of the present invention, the positive control antibody RG6058 used has a sequence that can be found in the sequence 34 and the sequence 36 in the Chinese Patent Publication No. CN108290946.

In the following examples of the present invention, the anti-CTLA4-anti-PD-1 bispecific antibody CP004(hG1TM) used in the combination was produced by Akeso Biopharma, Inc., the sequence of which can be found in Publication Patent CN112300286A, with reference to the full-length heavy chain sequence (an amino acid sequence set forth in SEQ ID NO: 57, and a nucleotide sequence set forth in SEQ ID NO: 58) of CP004(hG1TM) and the full-length light chain sequence (an amino acid sequence set forth in SEQ ID NO: 59, and a nucleotide sequence set forth in SEQ ID NO: 60) of CP004(hG1TM), and CP004(hG1TM) has a structure of IgG-scFv, wherein the IgG part is an anti-PD1 antibody, and the scFv part is an anti-CTLA4 antibody,
wherein the HCDR1 sequence of the anti-PD1 antibody is set forth in SEQ ID NO: 61, the HCDR2 sequence is set forth in SEQ ID NO: 62, the HCDR3 sequence is set forth in SEQ ID NO: 63, the VH sequence is set forth in SEQ ID NO: 73, the LCDR1 sequence of the anti-PD1 antibody is set forth in SEQ ID NO: 70, the LCDR2 sequence is set forth in SEQ ID NO: 71, the LCDR3 sequence is set forth in SEQ ID NO: 72, and the VL sequence is set forth in SEQ ID NO: 76;
the HCDR1 sequence of the anti-CTLA4 antibody is set forth in SEQ ID NO: 64, the HCDR2 sequence is set forth in SEQ ID NO: 65, the HCDR3 sequence is set forth in SEQ ID NO: 66, the VH sequence is set forth in SEQ ID NO: 74, the LCDR1 sequence of the anti-CTLA4 antibody is set forth in SEQ ID NO: 67, the LCDR2 sequence is set forth in SEQ ID NO: 68, the LCDR3 sequence is set forth in SEQ ID NO: 69, and the VL sequence is set forth in SEQ ID NO: 75.

### Example 1: Preparation of Anti-TIGIT Antibody 26B12

### 1. Preparation of hybridoma cell line LT019

The antigen used for preparing the anti-TIGIT antibody was human TIGIT-mFc (TIGIT was GenbankID: NP_776160.2; the mFc sequence was set forth in SEQ ID NO: 77). Spleen cells of immunized mice were fused with myeloma cells of the mice to prepare hybridoma cells. With human TIGIT-mFc taken as antigens, the hybridoma cells were screened by indirect ELISA to obtain hybridoma cells capable of secreting antibodies specifically binding to TIGIT. The hybridoma cells obtained by screening were subjected to limiting dilution to obtain a stable hybridoma cell line. The above hybridoma cell line was designated hybridoma cell line LT019, and the monoclonal antibody secreted by the cell line was designated 26B 12.

Hybridoma cell line LT019 (also called TIGIT-26B12) was deposited at China Center for Type Culture Collection (CCTCC) on Oct. 22, 2020, with an accession number of CCTCC NO: C2020208, the depository address being Wuhan University, Wuhan, China, postal code: 430072.

### 2. Preparation of anti-TIGIT antibody 26B12

The LT019 cell line prepared above was cultured in a chemical defined medium (CD medium, containing 1% penicillin-streptomycin) at 37 °C/5% CO₂. After 7 days, the cell culture supernatant was collected, subjected to high-speed centrifugation and vacuum filtration through a microfiltration membrane, and purified by using a HiTrap protein A HP column to obtain an antibody 26B12.

### Example 2: Sequence analysis of anti-TIGIT antibody 26B12

mRNA was extracted from the cell line LT019 cultured in Example 1 according to the method described in the manual of RNAprep pure Cell/Bacteria Kit (Tiangen, Cat. No. DP430).

cDNA was synthesized according to the manual of Invitrogen SuperScript^{®} III First-Strand Synthesis System for RT-PCR and amplified by PCR.

The PCR amplification products were directly subjected to TA cloning according to the manual of the pEASY-T1 Cloning Kit (Transgen CT101).

The TA cloning products were directly sequenced, and the sequencing results are as follows:
The nucleotide sequence of the heavy chain variable region is set forth in SEQ ID NO: 2 with a length of 363 bp.

The encoded amino acid sequence is set forth in SEQ ID NO: 1 with a length of 121 amino acids,
wherein the sequence of heavy chain HCDR1 is set forth in SEQ ID NO: 3, the sequence of HCDR2 is set forth in SEQ ID NO: 4, and the sequence of HCDR3 is set forth in SEQ ID NO: 5.

The nucleotide sequence of the light chain variable region is set forth in SEQ ID NO: 7 with a length of 321 bp.

The encoded amino acid sequence is set forth in SEQ ID NO: 6 with a length of 107 amino acids,
wherein the sequence of light chain LCDR1 is set forth in SEQ ID NO: 8, the sequence of LCDR2 is set forth in SEQ ID NO: 9, and the sequence of LCDR3 is set forth in SEQ ID NO: 10.

### Example 3: Design and preparation of light and heavy chains of humanized anti-human TIGIT antibodies

### 1. Design of light and heavy chains of humanized anti-human TIGIT antibodies 26B12H1L1, 26B12H4L1, 26B12H2L2, 26B12H3L2, 26B12H2L3, 26B12H3L3, 26B12H1L4, and 26B12H4L4

Based on the three-dimensional crystal structure of human TIGIT protein and the sequence of antibody 26B12 obtained in Example 2, the antibody model was simulated by computer, and mutations were designed according to the model to obtain the variable region sequences of antibodies 26B12H1L1, 26B12H4L1, 26B12H2L2, 26B12H3L2, 26B12H2L3, 26B12H3L3, 26B12H1L4, and 26B12H4L4 (antibody constant region sequences from the NCBI database: the heavy chain constant region is Ig gamma-1 chain C region, ACCESSION: P01857; the light chain constant region is Ig kappa chain C region, ACCESSION: P01834). The designed variable region sequences are shown in Table A below.

| No. | Name | Amino acid sequence of heavy chain variable region SEQ ID NO: | Nucleotide sequence of heavy chain variable region SEQ ID NO: | Amino acid sequence of light chain variable region SEQ ID NO: | Nucleotide sequence of light chain variable region SEQ ID NO: |
|---|---|---|---|---|---|
| 1 | 26B 12H1L1 | 11 | 12 | 19 | 20 |
| 2 | 26B12H4L1 | 17 | 18 | 19 | 20 |
| 3 | 26B12H2L2 | 13 | 14 | 21 | 22 |
| 4 | 26B12H2L3 | 13 | 14 | 23 | 24 |
| 5 | 26B12H3L2 | 15 | 16 | 21 | 22 |
| 6 | 26B12H3L3 | 15 | 16 | 23 | 24 |
| 7 | 26B12H1L4 | 11 | 12 | 25 | 26 |
| 8 | 26B12H4L4 | 17 | 18 | 25 | 26 |

### Heavy and light chain variable region sequences of humanized monoclonal antibody 26B12H1L4

For the above 8 antibodies 26B12H1L1, 26B12H4L1, 26B12H2L2, 26B12H3L2, 26B12H2L3, 26B12H3L3, 26B12H1L4, and 26B12H4L4, the length of the nucleotide sequences of the heavy chain variable regions was 363 bp, and the length of the encoded amino acid sequences was 121 aa; the length of the nucleotide sequences of the light chain variable regions was 321 bp, and the length of the encoded amino acid sequences was 107 aa.

Moreover, the above 8 antibodies had the same HCDR1-HCDR3 and LCDR1-LCDR3:
The sequence of HCDR1 is set forth in SEQ ID NO: 3, the sequence of HCDR2 is set forth in SEQ ID NO: 4, and the sequence of HCDR3 is set forth in SEQ ID NO: 5;
the sequence of LCDR1 is set forth in SEQ ID NO: 8, the sequence of LCDR2 is set forth in SEQ ID NO: 9, and the sequence of LCDR3 is set forth in SEQ ID NO: 10.

### 2. Preparation of humanized antibodies 26B12H1L1, 26B12H4L1, 26B12H2L2, 26B12H3L2, 26B12H2L3, 26B12H3L3, 26B12H1L4, and 26B12H4L4

The heavy chain constant regions were all Ig gamma-1 chain C region, ACCESSION: P01857; the light chain constant regions were all Ig kappa chain C region, ACCESSION: P01834.

Heavy chain cDNA and light chain cDNA of 26B12H1L1, heavy chain cDNA and light chain cDNA of 26B12H4L1, heavy chain cDNA and light chain cDNA of 26B12H2L2, heavy chain cDNA and light chain cDNA of 26B12H3L2, heavy chain cDNA and light chain cDNA of 26B12H2L3, heavy chain cDNA and light chain cDNA of 26B12H3L3, heavy chain cDNA and light chain cDNA of 26B12H1L4, heavy chain cDNA and light chain cDNA of 26B12H2L4, and heavy chain cDNA and light chain cDNA of 26B12H4L4 were separately cloned into pUC57simple (provided by Genscript) vectors to obtain pUC57simple-26B12H1, pUC57simple-26B12L1; pUC57simple-26B12H4, pUC57simple-26B12L1; pUC57simple-26B12H2, pUC57simple-26B12L2; pUC57simple-26B12H3, pUC57simple-26B12L2; pUC57simple-26B12H2, pUC57simple-26B12L3; pUC57simple-26B12H3, pUC57simple-26B12L3; pUC57simple-26B12H1, pUC57simple-26B12L4; pUC57simple-26B12H2, pUC57simple-26B12L4; and pUC57simple-26B12H4, pUC57simple-26B12L4, respectively. With reference to the standard techniques described in Molecular Cloning: A Laboratory Manual (Second Edition), the heavy and light chain full-length genes synthesized by EcoRI&HindIII digestion were subcloned into expression vector pcDNA3.1 by digestion with restriction enzyme EcoRI&HindIII to obtain expression plasmids pcDNA3.1-26B12H1, pcDNA3.1-26B12L1, pcDNA3.1-26B12H4, pcDNA3.1-126B12H2, pcDNA3.1-26B12L2, pcDNA3.1-26B12H3, pcDNA3.1-26B12L3 and pcDNA3.1-26B12L4, and the heavy/light chain genes of the recombinant expression plasmids were further sequenced. Then the designed gene combinations comprising corresponding light and heavy chain recombinant plasmids (pcDNA3.1- 26B12H1/pcDNA3.1-26B12L1, pcDNA3.1-26B12H4/pcDNA3.1-26B12L1, pcDNA3.1-26B12H2/pcDNA3.1-26B12L2, pcDNA3.1-26B12H3/ pcDNA3.1-26B12L2, pcDNA3.1-26B12H2/pcDNA3.1-26B12L3, pcDNA3.1-26B12H3/pcDNA3.1-26B12L3, pcDNA3.1-26B12H1/pcDNA3.1-26B12L4 and pcDNA3.1-26B12H4/pcDNA3.1-26B12L4) were separately co-transfected into 293F cells, and the cultures were collected and purified. After the sequences were verified, endotoxin-free expression plasmids were prepared, and were transiently transfected into HEK293 cells for antibody expression. After 7 days, the cell cultures were collected and subjected to affinity purification on a Protein A column to obtain humanized antibodies.

### Example 4: ELISA Assays for Activity of Antibodies Binding to Antigen TIGIT-mFc

Experimental steps: Goat anti-mouse IgG Fc (purchased from Jackson, lot no. 132560) was added at 2 µg/mL to coat a microplate and incubated at 4 °C for 16 h. After incubation, the microplate coated with the goat anti-mouse IgG Fc was washed once with PBST, and then blocked with a PBST solution containing 1% BSA as a microplate blocking solution for 2 h. After blocking, the microplate was washed 3 times with PBST. Then, 1 µg/mL antigen human TIGIT-mFc was added, and the plate was incubated at 37 °C for 30 min and then washed 3 times with PBST. The antibodies serially diluted with the PBST solution were added to the wells of the microplate. The antibody dilution gradients are shown in Table 1 and Table 2. The microplate containing the test antibodies was incubated at 37 °C for 30 min, and then washed 3 times with PBST. After washing, HRP-labeled goat anti-human IgG Fc (purchased from Jackson, lot no. 128332) secondary antibody working solution diluted in a ratio of 1:5000 was added, and the microplate was incubated at 37 °C for 30 min. After incubation, the plate was washed 4 times with PBST, TMB (Neogen, 308177) was added for chromogenesis in the dark for 4 min, and then a stop solution was added to terminate the chromogenic reaction. The microplate was put into a microplate reader immediately, and the OD value of each well in the microplate was read at 450 nm. The data were analyzed and processed by SoftMax Pro 6.2.1.

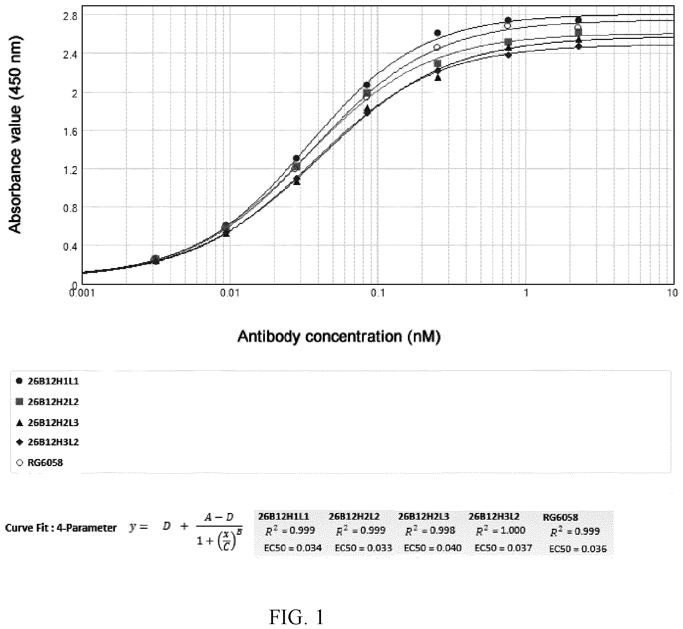

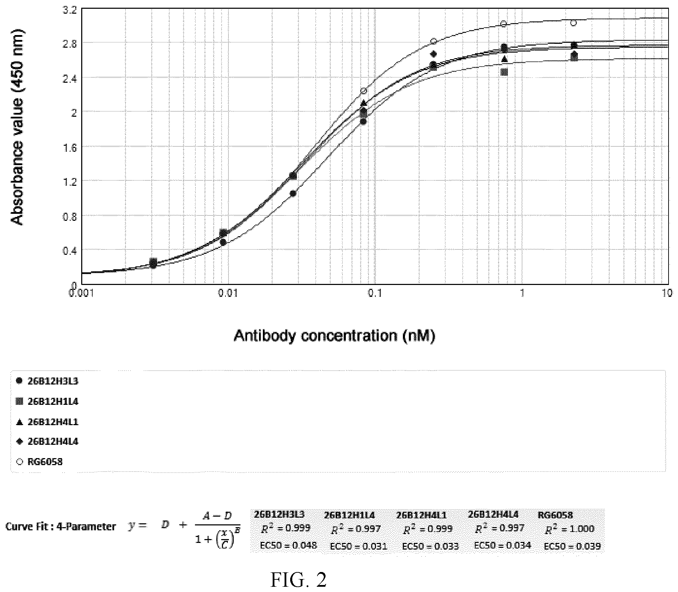

The results of the binding of antibody to antigen TIGIT-mFc are shown in FIG. 1 and FIG. 2. The OD values for all the dosages are shown in Table 1 and Table 2. The EC₅₀ of the antibodies binding to the antigen was calculated by curve fitting using antibody concentration as the abscissa and absorbance value as the ordinate. The results are shown in Table 1 and Table 2, and FIG. 1 and FIG. 2.

**Table 1: The results of assays for the activity of 26B12H1L1, 26B12H2L2, 26B12H2L3, 26B12H3L2 and RG6058 binding to TIGIT-mFc**

| Coating: goat anti-mouse IgG Fc (2 µg/mL) | | | | | | | | | | |
|---|---|---|---|---|---|---|---|---|---|---|
| Antibody dilution (µg/mL) | TIGIT-mFc (1µg/mL) | | | | | | | | | |
| | 26B12H1L1 | | 26B12H2L2 | | 26B12H2L3 | | 26B12H3L2 | | RG6058 | |
| 0.3330 | 2.789 | 2.701 | 2.623 | 2.609 | 2.560 | 2.542 | 2.513 | 2.430 | 2.644 | 2.689 |
| 0.1110 | 2.722 | 2.771 | 2.460 | 2.572 | 2.413 | 2.528 | 2.368 | 2.394 | 2.516 | 2.863 |
| 0.0370 | 2.539 | 2.676 | 2.322 | 2.253 | 2.162 | 2.137 | 2.142 | 2.292 | 2.332 | 2.605 |
| 0.0123 | 2.001 | 2.126 | 2.001 | 1.965 | 1.853 | 1.811 | 1.706 | 1.861 | 1.855 | 2.028 |
| 0.0041 | 1.262 | 1.349 | 1.211 | 1.221 | 1.038 | 1.103 | 1.106 | 1.092 | 1.142 | 1.263 |
| 0.0014 | 0.593 | 0.613 | 0.542 | 0.627 | 0.506 | 0.544 | 0.546 | 0.527 | 0.569 | 0.602 |
| 0.0005 | 0.264 | 0.265 | 0.249 | 0.258 | 0.237 | 0.243 | 0.224 | 0.229 | 0.238 | 0.254 |
| 0.0000 | 0.056 | 0.051 | 0.053 | 0.046 | 0.052 | 0.049 | 0.049 | 0.050 | 0.053 | 0.049 |
| Secondary antibody | HRP-labeled goat anti-human IgG Fc (1:5000) | | | | | | | | | |
| EC₅₀ (nM) | 0.034 | 0.033 | 0.040 | 0.037 | 0.036 | | | | | |

**Table 2: The results of assays for the activity of 26B12H3L3, 26B12H1L4, 26B12H4L1, 26B12H4L4 and RG6058 binding to TIGIT-mFc**

| Coating: goat anti-mouse IgG Fc (2 µg/mL) | | | | | | | | | | |
|---|---|---|---|---|---|---|---|---|---|---|
| Antibody dilution (µg/mL) | TIGIT-mFc (1µg/mL) | | | | | | | | | |
| | 26B12H3L3 | | 26B12H1L4 | | 26B12H4L1 | | 26B12H4L4 | | RG6058 | |
| 0.3330 | 2.736 | 2.788 | 2.639 | 2.604 | 2.709 | 2.829 | 2.728 | 2.608 | 2.963 | 3.089 |
| 0.1110 | 2.707 | 2.774 | 2.469 | 2.422 | 2.625 | 2.587 | 2.626 | 2.788 | 2.915 | 3.119 |
| 0.0370 | 2.546 | 2.538 | 2.568 | 2.451 | 2.392 | 2.699 | 2.679 | 2.660 | 2.830 | 2.797 |
| 0.0123 | 1.861 | 1.881 | 2.049 | 1.882 | 2.113 | 2.091 | 1.987 | 2.045 | 2.237 | 2.237 |
| 0.0041 | 1.074 | 1.012 | 1.232 | 1.266 | 1.252 | 1.279 | 1.265 | 1.239 | 1.254 | 1.258 |
| 0.0014 | 0.483 | 0.477 | 0.593 | 0.580 | 0.582 | 0.592 | 0.589 | 0.593 | 0.569 | 0.593 |
| 0.0005 | 0.217 | 0.211 | 0.246 | 0.263 | 0.256 | 0.261 | 0.253 | 0.253 | 0.244 | 0.248 |
| 0.0000 | 0.065 | 0.060 | 0.053 | 0.051 | 0.051 | 0.051 | 0.052 | 0.054 | 0.065 | 0.061 |

| Secondary antibody | HRP-labeled goat anti-human IgG Fc (1:5000) | | | | | | | | | |
|---|---|---|---|---|---|---|---|---|---|---|
| EC₅₀ (nM) | 0.048 | | 0.031 | | 0.033 | | 0.034 | | 0.039 | |

The results show that the antibodies 26B12H1L1, 26B12H4L1, 26B12H2L2, 26B12H3L2, 26B12H2L3, 26B12H3L3, 26B12H1L4 and 26B12H4L4 were all capable of effectively binding to human TIGIT-mFc in a dose-dependent manner, and the binding activities were comparable to that of the positive drug RG6058 for the same target, indicating that the antibodies 26B12H1L1, 26B12H4L1, 26B12H2L2, 26B12H3L2, 26B12H2L3, 26B12H3L3, 26B12H1L4 and 26B12H4L4 had the function of effectively binding to TIGIT.

### Example 5: Competitive ELISA Assays for Activity of Antibodies Competing with CD155-hFc-Biotin for Binding to TIGIT-mFc

Experimental steps: A microplate was coated with TIGIT-mFc at 2 µg/mL, and incubated at 4 °C overnight. After incubation, the antigen-coated microplate was rinsed once with PBST, and then blocked with a PBST solution containing 1% BSA as a microplate blocking solution for 2 h. After blocking, the microplate was washed 3 times with PBST. The antibodies serially diluted with the PBST solution were added to the microplate. The antibody concentrations are shown in Table 3 and Table 4. After the microplate was incubated at room temperature for 10 min, equal volumes of 2 µg/mL CD155-hFc-Biotin (produced by Akeso Biopharma, Inc., lot no. 20170210, wherein CD155 was GenBank accession no. NP_006496.4, and the hFc sequence is set forth in SEQ ID NO: 78; final concentration: 1 µg/mL) were added, and mixed well with the antibodies. The microplate was incubated at 37 °C for 30 min, and then washed 3 times with PBST. After washing, an SA-HRP working solution diluted in a ratio of 1:4000 was added, and the microplate was incubated at 37 °C for 30 min. After incubation, the plate was washed 4 times with PBST, TMB (Neogen, 308177) was added for chromogenesis in the dark for 5 min, and then a stop solution was added to terminate the chromogenic reaction. The microplate was put into a microplate reader immediately, and the OD value of each well in the microplate was read at 450 nm. The data were analyzed and processed by SoftMax Pro 6.2.1.

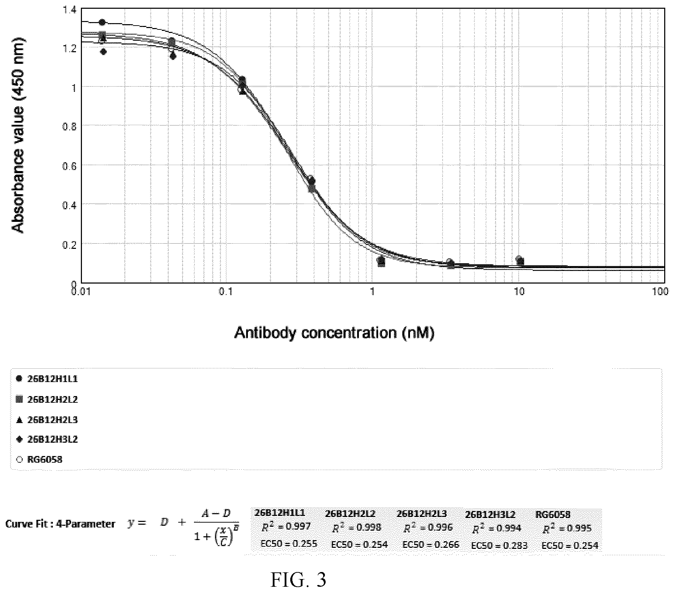

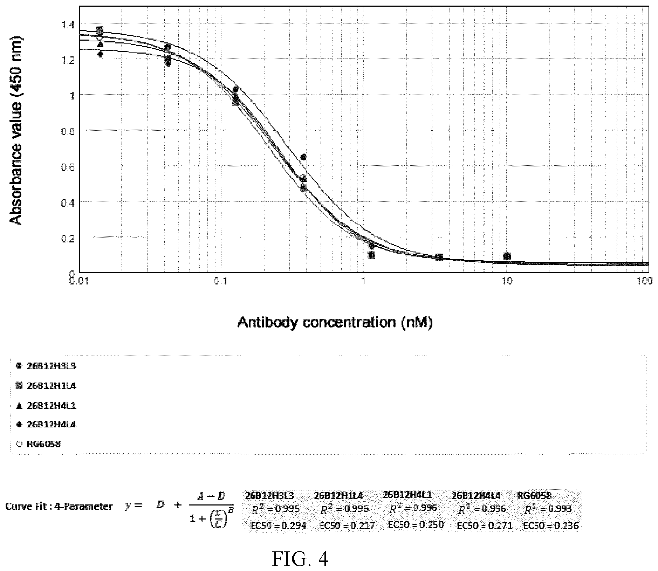

The results of the activity of the antibodies competing with CD155-hFc-Biotin for binding to TIGIT-mFc are shown in Table 3 and Table 4. The EC₅₀ of the antibodies competing with CD155-hFc-Biotin for binding to TIGIT-mFc was calculated by curve fitting using antibody concentration as the abscissa and absorbance value as the ordinate, and the results are shown in Table 3 and Table 4, and FIG. 3 and FIG. 4 below.

**Table 3: The results of assays for the activity of 26B12H1L1, 26B12H2L2, 26B12H2L3, 26B12H3L2 and RG6058 competing with CD155-hFc-Biotin for binding to TIGIT-mFc**

| Antibody dilution | Antigen coating: TIGIT-mFc (2 µg/mL) | | | | | | | | | |
|---|---|---|---|---|---|---|---|---|---|---|
| | 26B12H1L1 | | 26B12H2L2 | | 26B12H2L3 | | 26B12H3L2 | | RG6058 | |
| 3 µg/mL | 0.091 | 0.097 | 0.101 | 0.110 | 0.106 | 0.117 | 0.107 | 0.113 | 0.119 | 0.120 |
| 1:3 | 0.088 | 0.085 | 0.082 | 0.092 | 0.098 | 0.100 | 0.101 | 0.102 | 0.104 | 0.112 |
| 1:9 | 0.104 | 0.099 | 0.091 | 0.097 | 0.107 | 0.110 | 0.121 | 0.120 | 0.114 | 0.121 |
| 1:27 | 0.533 | 0.491 | 0.410 | 0.538 | 0.510 | 0.537 | 0.492 | 0.549 | 0.528 | 0.532 |
| 1:81 | 1.026 | 1.035 | 0.996 | 1.025 | 0.961 | 0.990 | 0.948 | 1.059 | 0.951 | 1.011 |
| 1:243 | 1.210 | 1.251 | 1.222 | 1.221 | 1.142 | 1.195 | 1.089 | 1.217 | 1.168 | 1.209 |
| 1:729 | 1.287 | 1.360 | 1.274 | 1.242 | 1.201 | 1.287 | 1.120 | 1.236 | 1.209 | 1.251 |
| 0 | 1.347 | 1.387 | 1.315 | 1.296 | 1.279 | 1.307 | 1.263 | 1.340 | 1.295 | 1.354 |

| CD155-hFc-Biotin (1µg/mL) | | | | | | | | | | |
|---|---|---|---|---|---|---|---|---|---|---|
| SA-HRP (1:4000) | | | | | | | | | | |
| EC₅₀ (nM) | 0.255 | | 0.254 | | 0.266 | | 0.283 | | 0.254 | |

**Table 4: The results of assays for the activity of 26B12H3L3, 26B12H1L4, 26B12H4L1, 26B12H4L4 and RG6058 competing with CD155-hFc-Biotin for binding to TIGIT-mFc**

| Antibody dilution | Antigen coating: TIGIT-mFc (2 µg/mL) | | | | | | | | | |
|---|---|---|---|---|---|---|---|---|---|---|
| | 26B12H3L3 | | 26B12H1L4 | | 26B12H4L1 | | 26B12H4L4 | | RG6058 | |
| 3 µg/mL | 0.087 | 0.085 | 0.091 | 0.084 | 0.102 | 0.086 | 0.091 | 0.085 | 0.091 | 0.093 |
| 1:3 | 0.083 | 0.082 | 0.077 | 0.086 | 0.086 | 0.086 | 0.084 | 0.082 | 0.089 | 0.084 |
| 1:9 | 0.139 | 0.146 | 0.094 | 0.096 | 0.106 | 0.102 | 0.103 | 0.103 | 0.101 | 0.100 |
| 1:27 | 0.646 | 0.650 | 0.449 | 0.495 | 0.495 | 0.561 | 0.516 | 0.532 | 0.530 | 0.539 |
| 1:81 | 1.031 | 1.027 | 0.938 | 0.967 | 0.931 | 1.030 | 0.974 | 0.999 | 0.946 | 1.037 |
| 1:243 | 1.239 | 1.294 | 1.171 | 1.224 | 1.180 | 1.232 | 1.131 | 1.218 | 1.152 | 1.223 |
| 1:729 | 1.318 | 1.378 | 1.336 | 1.382 | 1.278 | 1.292 | 1.123 | 1.335 | 1.302 | 1.346 |
| 0 | 1.410 | 1.393 | 1.341 | 1.361 | 1.357 | 1.360 | 1.258 | 1.364 | 1.380 | 1.427 |

| CD155-hFc-Biotin (1µg/mL) | | | | | | | | | | |
|---|---|---|---|---|---|---|---|---|---|---|
| SA-HRP (1:4000) | | | | | | | | | | |
| EC₅₀ (nM) | 0.294 | 0.217 | 0.250 | 0.271 | 0.236 | | | | | |

The results show that in the same experimental conditions, 26B12H1L1, 26B12H4L1, 26B12H2L2, 26B12H3L2, 26B12H2L3, 26B12H3L3, 26B12H1L4 and 26B12H4L4 could compete with CD155-hFc-Biotin for binding to the antigen TIGIT-mFc, and the activities were comparable to that of the positive drug RG6058 for the same target, indicating that 26B12H1L1, 26B12H4L1, 26B12H2L2, 26B12H3L2, 26B12H2L3, 26B12H3L3, 26B12H1L4 and 26B12H4L4 had an effective function of competing with CD155-hFc-Biotin for binding to TIGIT-mFc.

### Example 6: Assays for Kinetic Parameters of Humanized Antibodies 26B12H3L3, 26B12H1L1. 26B12H2L2. 26B12H2L3. 26B12H3L2. 26B12H4L4, 26B12H1L4, 26B12H4L1 and RG6058 Binding to Antigen TIGIT-mFc Using Fortebio Molecular Interaction Instrument

The sample dilution buffer was PBS (0.02% Tween-20, 0.1% BSA, pH 7.4). TIGIT-mFc was immobilized on an AMC sensor at a concentration of 3 µg/mL for 50 s, and the sensor was equilibrated in the buffer for 60 s. The TIGIT-mFc immobilized on the sensor was allowed to bind to the antibody at concentrations of 0.06-5 nM (three-fold dilutions) for 120 s, and the protein was dissociated in the buffer for 300 s. The sensor was regenerated with 10 mM glycine solution (pH = 1.7). The detection temperature was 37 °C, the detection frequency was 0.3 Hz, and the sample plate shaking rate was 1000 rpm. The data were analyzed by 1:1 model fitting to obtain affinity constants.

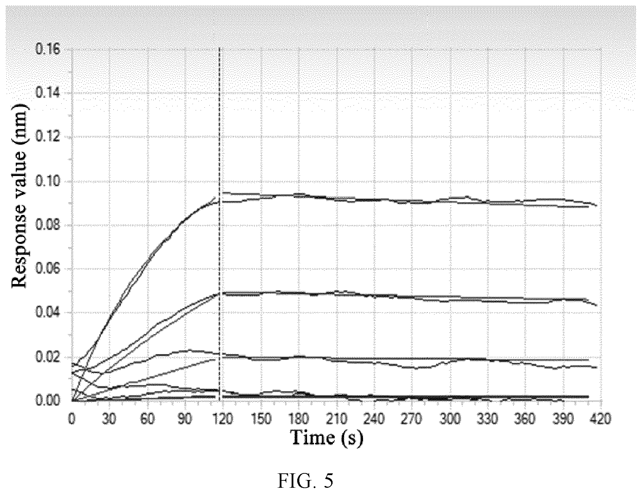

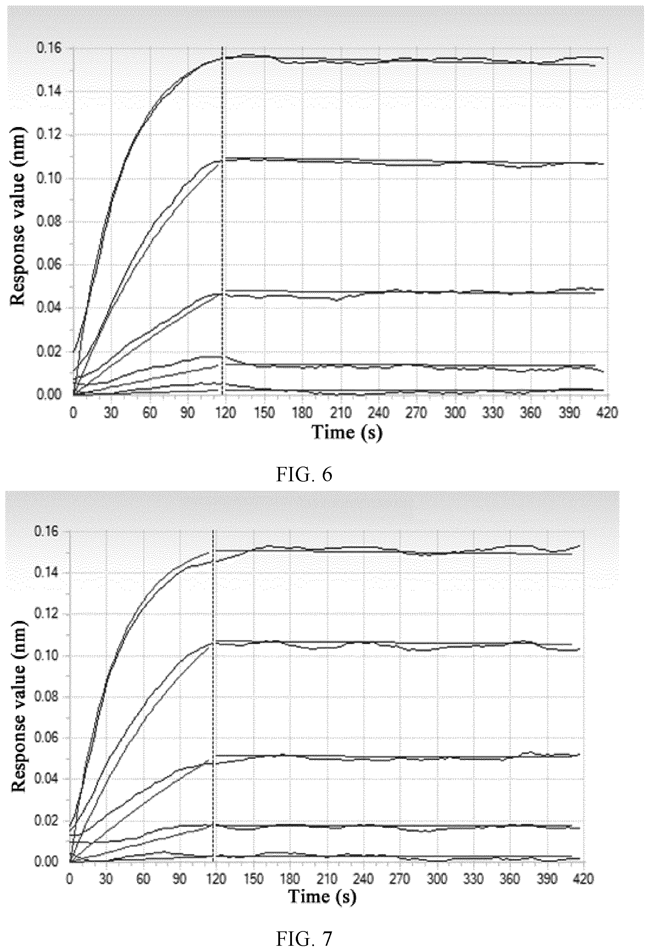

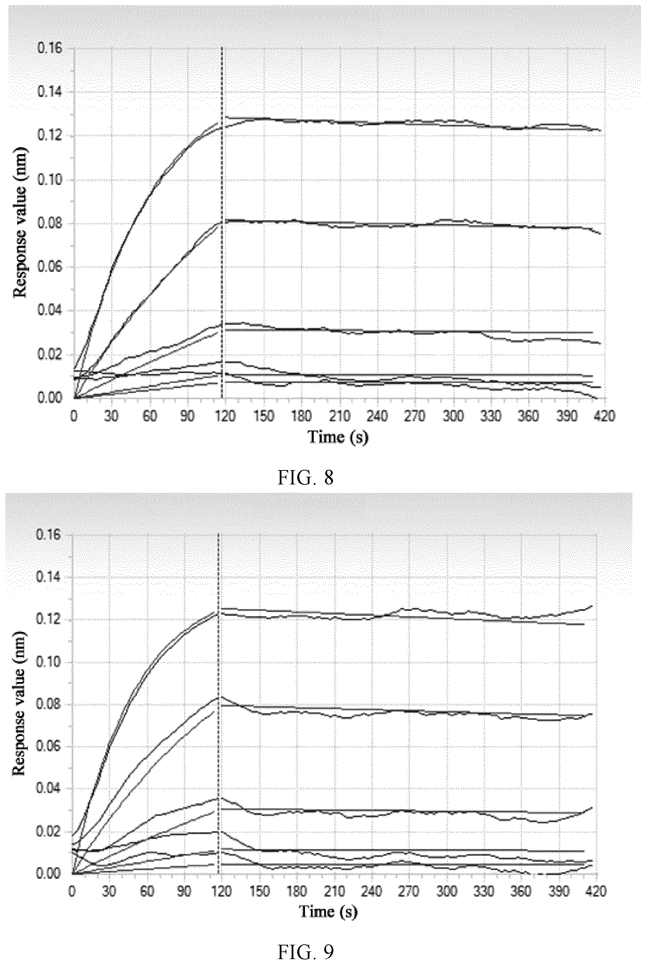

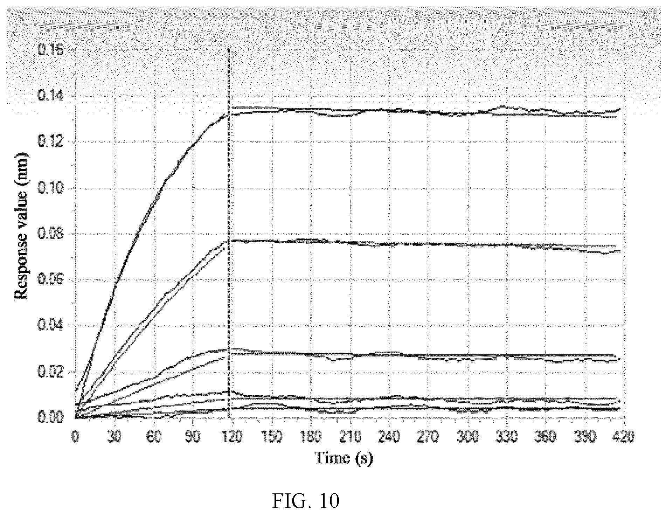

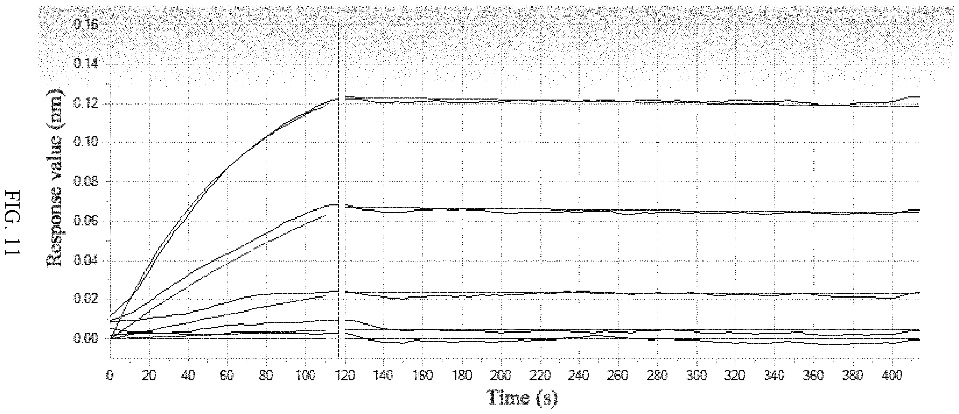

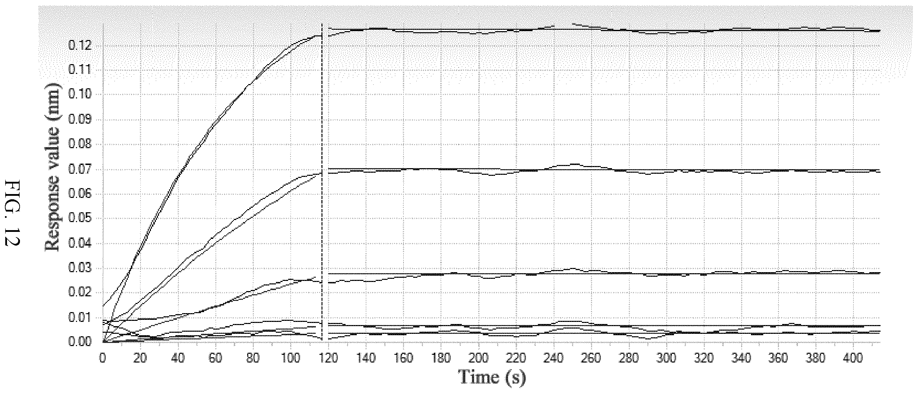

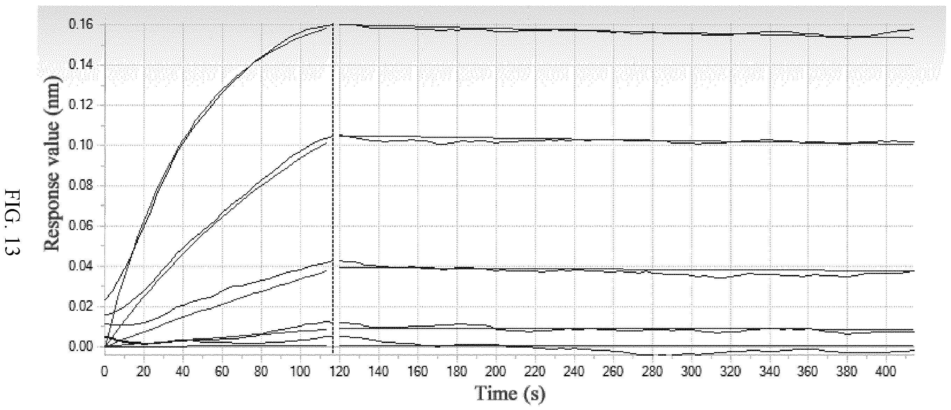

The results of assays for the affinity constants of the humanized antibodies (as control antibody) for TIGIT are shown in Table 5 and FIGs. 5-13.

**Table 5: The results of assays for the affinity constants of humanized antibodies for antigen TIGIT-mFc**

| Antibody | Maximum | K_{D} (M) | Kon | S E (kon) | Kdis (1/s) | S E (kdis) | Rmax |
|---|---|---|---|---|---|---|---|
| | signal height of analyte (nm) | | (1/Ms) | | | | (nm) |
| 26B12H3L3 | 0.09 | 9.64E-11 | 2.48E+06 | 3.22E+05 | 2.39E-04 | 8.57E-05 | 0.05-0.13 |
| 26B12H1L1 | 0.15 | 1.64E-11 | 5.44E+06 | 1.92E+05 | 8.93E-05 | 3.36E-05 | 0.06-0.17 |
| 26B12H2L2 | 0.14 | 8.40E-12 | 5.47E+06 | 2.55E+05 | 4.60E-05 | 4.40E-05 | 0.07-0.17 |
| 26B12H2L3 | 0.12 | 4.85E-11 | 3.29E+06 | 2.53E+05 | 1.59E-04 | 5.92E-05 | 0.15-0.31 |
| 26B12H3L2 | 0.12 | 5.40E-11 | 3.94E+06 | 3.60E+05 | 2.13E-04 | 7.86E-05 | 0.13-0.17 |
| 26B12H4L4 | 0.13 | 3.69E-11 | 2.81E+06 | 1.38E+05 | 1.04E-04 | 3.23E-05 | 0.14-0.20 |
| 26B12H1L4 | 0.12 | 4.63E-11 | 2.94E+06 | 1.96E+05 | 1.36E-04 | 4.88E-05 | 0.01-0.15 |
| 26B12H4L1 | 0.12 | 8.57E-12 | 2.90E+06 | 1.34E+05 | 2.48E-05 | 3.11E-05 | 0.11-0.18 |
| RG6058 | 0.16 | 3.16E-11 | 4.56E+06 | 1.84E+05 | 1.44E-04 | 3.46E-05 | 0.01-0.18 |

K_{D} is the affinity constant; K_{D} = kdis/kon.

The results show that the affinity constants of the humanized antibodies 26B12H3L3, 26B12H1L1, 26B12H2L2, 26B12H2L3, 26B12H3L2, 26B12H4L4, 26B12H1L4, 26B12H4L1 and RG6058 for TIGIT-mFc were 9.64E-11M, 1.64E-11M, 8.40E-12M, 4.85E-11M, 5.40E-11M, 3.69E-11M, 4.63E-11M, 8.57E-12M and 3.16E-11M, respectively.

The results demonstrate that according to the level of affinity for TIGIT-mFc, the antibodies against TIGIT could be listed in descending order as follows: 26B12H2L2, 26B12H4L1, 26B12H1L1, RG6058, 26B12H4L4, 26B12H1L4, 26B12H2L3, 26B12H3L2, and 26B12H3L3. Among them, the humanized antibodies 26B12H2L2, 26B12H4L1 and 26B12H1L1 have stronger affinity than the positive drug RG6058, and 26B12H4L4 has a comparable affinity to the positive drug RG6058.

Example 7: FACS Assays for the Activity of Humanized Antibodies 26B12H2L2 and RG6058 Binding to 293T-TIGIT Cell Membrane Surface Antigen TIGIT Experimental method:
TIGIT vector plenti6.3-TIGITFL-BSD (TIGIT was GenbankID: NP_776160.2; human TIGIT full-length cDNA sequence was optimized and synthesized by GenScript, designated as TIGITFL, and cloned into the pUC57simple vector (supplied by GenScript) to give the pUC57simple-TIGITFL plasmid. The pUC57simple-TIGITFL plasmid was synthesized with BamHI and XhoI dual enzyme digestion; the TIGITFL target gene fragment was collected and subcloned into a plenti6.3 expression vector through restriction sites BamHI and XhoI; the vector pLenti6.3 (purchased from Invitrogen) was used to transfect 293T cells, and the cell line 293T-TIGIT stably expressing TIGIT was obtained by screening.

293T-TIGIT cells were collected (DMEM + 10% FBS) and centrifuged for 5 min, and the supernatant was discarded. The cells were resuspended and counted, and the cell viability was determined (P7, 95.79%). The cells were diluted, 300 thousand cells were added to each well of a transparent V-bottomed 96-well plate, and 200 µL of 1% PBSA was added to each tube. The mixtures were centrifuged for 5 min, and the supernatants were discarded. According to the experimental design, 100 µL of antibodies were added to each well (final concentration: 300 nM, 100 nM, 33.3 nM, 11.1 nM, 3.7 nM, 1.23 nM, 0.41 nM, 0.041 nM, and 0.0041 nM), and blank control and isotype control were designed. The plate was incubated on ice for 60 min. 200 µL of 1% PBSA was added to each tube. The mixtures were centrifuged for 5 min, and the supernatants were discarded. The plate was washed twice. The FITC goat anti-human IgG antibody (purchased from Jackson, Cat. No. 109-095-098, diluted 500-fold with PBSA) was added to each sample, and the mixtures were incubated on ice in the dark for 40 min. 200 µL of PBSA was added to each tube. The mixtures were centrifuged for 5 min, and the supernatants were discarded. The cells were resuspended by adding 200 µL of PBSA, and the suspensions were transferred into flow cytometry tubes to measure the mean fluorescence intensity of the cells at each concentration by a flow cytometer.

**Table 6: FACS assays for the activity of humanized antibodies 26B12H2L2 and RG6058 binding to 293T-TIGIT cell membrane surface antigen TIGIT**

| Antibody/ concentration (nM) | Mean fluorescence intensity | | | | | | | | | EC₅₀ (nM) |
|---|---|---|---|---|---|---|---|---|---|---|
| | 300 | 100 | 33.33 | 11.1 | 3.7 | 1.23 | 0.41 | 0.041 | 0.0041 | |
| RG6058 | 505.61 | 554.87 | 493.98 | 537.75 | 431.36 | 266.73 | 109.14 | 24.47 | 14.61 | 1.257 |
| 26B12H2L2 | 514.58 | 467.29 | 412.32 | 645.99 | 466.99 | 320.40 | 122.58 | 28.76 | 12.66 | 0.917 |

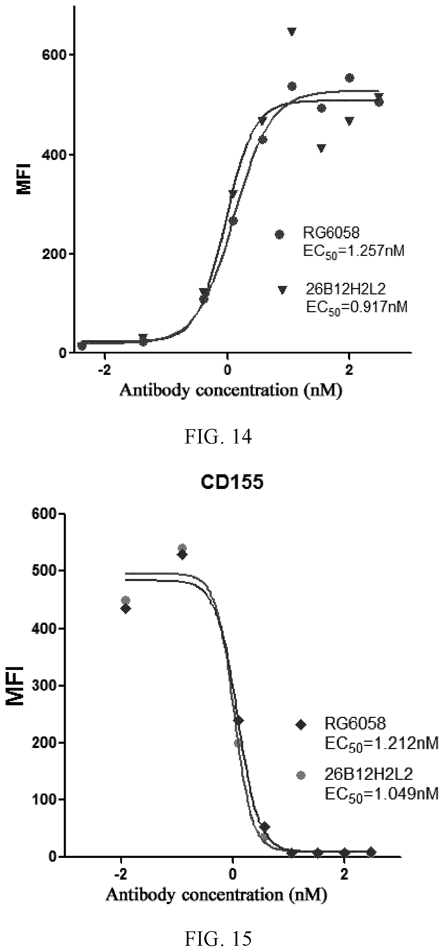

The experimental results are shown in Table 6 and FIG. 14. The EC₅₀ for binding of the positive control antibody RG6058 to the cell membrane surface antigen TIGIT was 1.257 nM, and the EC₅₀ for binding of the humanized antibody 26B12H2L2 to the cell membrane surface antigen TIGIT was 0.917 nM.

The experimental results demonstrate that the humanized antibody 26B12H2L2 had a stronger binding ability to the cell membrane surface antigen TIGIT than the positive control antibody RG6058.

### Example 8: FACS Assays for the Activity of Humanized Antibodies 26B12H2L2 and RG6058 Competing with CD155 or CD112 for Binding to 293T-TIGIT Cell Membrane Surface TIGIT

Experimental method: 293T-TIGIT cells were collected and centrifuged for 5 min, and the supernatant was discarded. The cells were resuspended and counted, and the cell viability was determined (94.95%). The cells were diluted, 300 thousand cells were added to each well of a transparent V-bottomed 96-well plate, and 200 µL of 1% PBSA was added to each tube. The mixtures were centrifuged for 5 min, and the supernatants were discarded. According to the experimental design, 100 µL of antibodies were added to each well (final concentration: 300 nM, 100 nM, 33.3 nM, 11.1 nM, 3.7 nM, 1.23 nM, 0.123 nM, and 0.0123 nM), and blank control and isotype control were designed. The plate was incubated on ice for 30 min. CD155 (final concentration: 10 nM; produced by Akeso Biopharma, Inc., lot no. 20190726, wherein CD155 was GenBank accession no. NP_006496.4) or CD112 (final concentration: 30 nM; produced by Akeso Biopharma, Inc., lot no. 20190726, wherein CD112 was GenBank accession no. NP_001036189.1) was added to each sample, and the mixtures were incubated on ice in the dark for 60 min. 200 µL of 1% PBSA was then added to each tube. The mixtures were centrifuged for 5 min, and the supernatants were discarded. The plate was washed twice. The APC goat anti-mouse IgG antibody (purchased from Biolegend, lot no. 405308, minimal x-reactivity; diluted 300-fold with PBSA) was added to each sample, and the mixtures were incubated on ice in the dark for 40 min. 200 µL of PBSA was added to each tube. The mixtures were centrifuged for 5 min, and the supernatants were discarded. The cells were resuspended by adding 200 µL of PBSA, and the suspensions were transferred into flow cytometry tubes to measure the mean fluorescence intensity of the cells at each concentration by a flow cytometer.

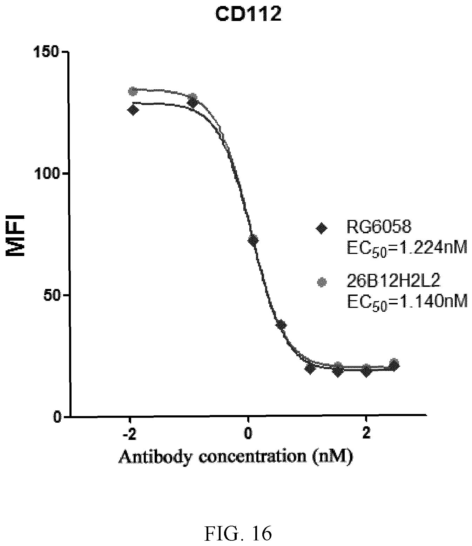

The experimental results are shown in Table 7 and FIG. 15, as well as Table 8 and FIG. 16.

**Table 7: FACS assays for the activity of humanized antibodies 26B12H2L2 and RG6058 competing with CD155 for binding to 293T-TIGIT cell membrane surface TIGIT**

| Antibody/ concentration (nM) | Mean fluorescence intensity | | | | | | | | EC₅₀ (nM) |
|---|---|---|---|---|---|---|---|---|---|
| | 300 | 100 | 33.3 | 11.1 | 3.7 | 1.23 | 0.123 | 0.0123 | |
| RG6058 | 8.85 | 7.44 | 7.6 | 7.71 | 52.1 | 239 | 530 | 436 | 1.212 |
| 26B12H2L2 | 8.3 | 7.64 | 7.83 | 8.2 | 36.1 | 200 | 541 | 449 | 1.049 |

**Table 8: FACS assays for the activity of humanized antibodies 26B12H2L2 and RG6058 competing with CD112 for binding to 293T-TIGIT cell membrane surface TIGIT**

| Antibody/ concentration (nM) | Mean fluorescence intensity | | | | | | | | EC₅₀ (nM) |
|---|---|---|---|---|---|---|---|---|---|
| | 300 | 100 | 33.3 | 11.1 | 3.7 | 1.23 | 0.123 | 0.0123 | |
| RG6058 | 20.1 | 17.8 | 18 | 19.2 | 37.1 | 72 | 129 | 126 | 1.224 |
| 26B12H2L2 | 21.4 | 19.2 | 20.3 | 19.8 | 37.1 | 73.1 | 131 | 134 | 1.140 |

The results show that the EC₅₀ of the positive control antibody RG6058 competing with CD155 for binding to TIGIT was 1.212 nM, and the EC₅₀ of the humanized antibody 26B12H2L2 competing with CD155 for binding to TIGIT was 1.049 nM; the EC₅₀ of the positive control antibody RG6058 competing with CD112 for binding to TIGIT was 1.224 nM, and the EC₅₀ of the humanized antibody 26B12H2L2 competing with CD112 for binding to TIGIT was 1.140 nM.

The experimental results demonstrate that the humanized antibody 26B12H2L2 had a stronger ability to compete with CD155 or CD112 for binding to the cell membrane surface antigen TIGIT than the positive control antibody RG6058.

### Example 9: Mixed Lymphocyte Reaction After Adding TIGIT Antibodies to Jurkat-TIGIT and HT1080-aCD3scFv Cell Systems

### Experimental method:

Transfecting Jurkat cells by using a TIGIT vector plenti6.3/V5-TIGITFL-BSD (the vector pLenti6.3 was purchased from Invitrogen), and screening to obtain a cell line of Jurkat-TIGIT cells stably expressing TIGIT; anti-CD3 antibody vector pCDH-aCD3scFv-puro (the cDNA sequence of the anti-CD3scFv was optimized and synthesized by GenScript, and cloned into the pUC57simple vector (supplied by GenScript) to give the pUC57simple-anti-CD3scFv plasmid. The synthesized pUC57simple-anti-CD3scFv plasmid was digested with XbaI and BamHI dual enzyme, the anti-CD3scFv target gene fragment was collected and subcloned into pCDH-CMV-MCS-EF1-Puro (purchased from Youbio) expression vector via restriction sites XbaI and BamHI (anti-CD3scFv sequence was derived from reference: Eukaryotic expression of anti-CD3 single chain Fv antibody gene and the characterization of its bioactivities JOURNAL Xi Bao Yu Fen Zi Mian Yi Xue Za Zhi 20 (5), 552-555 (2004), PUBMED 15367345); HT-1080 cells were transfected, and cell lines HT1080-aCD3scFv cells which stably express anti-CD3scFv on cell membranes were obtained through screening.

Jurkat-TIGIT and HT1080-aCD3scFv cells in logarithmic growth phase were collected. In 96-well plates, 50 thousand Jurkat-TIGIT cells were added to each well, and 10 thousand HT1080-aCD3scFV cells were added to each well. The diluted antibodies (final concentration: 10 nM, 50 nM, and 250 nM) and anti-human CD28 antibody (purchased from R&D, Cat. No. MAB342-500, 3 µg/mL) were added. The plates were incubated in an incubator for 48 h. The culture supernatants were collected and assayed for IL-2 content using IL-2 ELISA kit.

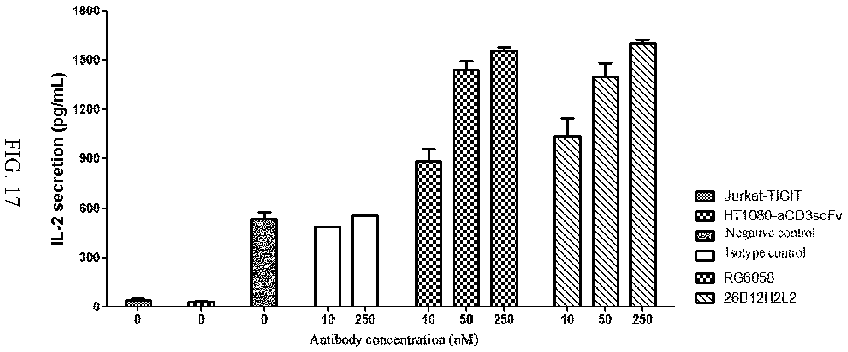

The experimental results are shown in FIG. 17.

The results show that the humanized antibody 26B12H2L2 and the positive control antibody RG6058 could both promote IL-2 secretion in the systems, and the humanized antibody 26B12H2L2 promoted IL-2 secretion to similar levels to RG6058 at these concentrations (10 nM, 50 nM, and 250 nM).

The results demonstrate that the ability of the humanized antibody 26B12H2L2 to induce the IL-2 secretion of the cells was comparable to that of the positive control antibody RG6058.

### Example 10: Therapeutic Effect of 26B 12H2L2 on CT26 Mouse Xenograft Tumors in hTigit-BALB/c Transgenic Mice

hTigit-BALB/c transgenic mice (purchased from GemPharmatech Co., Ltd, in which the normal TIGIT gene was replaced by the human TIGIT gene) were each inoculated with 500 thousand CT26 cells (mouse colon cancer cell line, purchased from ATCC) in the back. Specifically, a mouse tumor model was established by inoculating 200 µL of 25 million/mL CT26 cells into each mouse. The mice were divided into groups of 8 mice, including an isotype control group (20 mg/kg, i.p., twice weekly) and a treatment group (20 mg/kg, i.p., twice weekly). The specific regimen is shown in Table 9.

**Table 9: Establishment of mouse CT26 tumor model and administration regimen of antibodies**

| Group | Number of cells | Number of animals | Modeling | Administration regimen |
|---|---|---|---|---|
| Isotype control | 500 thousand cells/mouse | 8 | CT26 cells: 25 million cells/mL | hIgG1 20 mg/kg, Intraperitoneal injection (i.p.), twice weekly |
| 26B12H2L2 | 500 thousand cells/mouse | 8 | Inoculation volume: 200 µL/mouse | 26B12H2L2 20 mg/kg, intraperitoneal injection (i.p.), twice weekly |

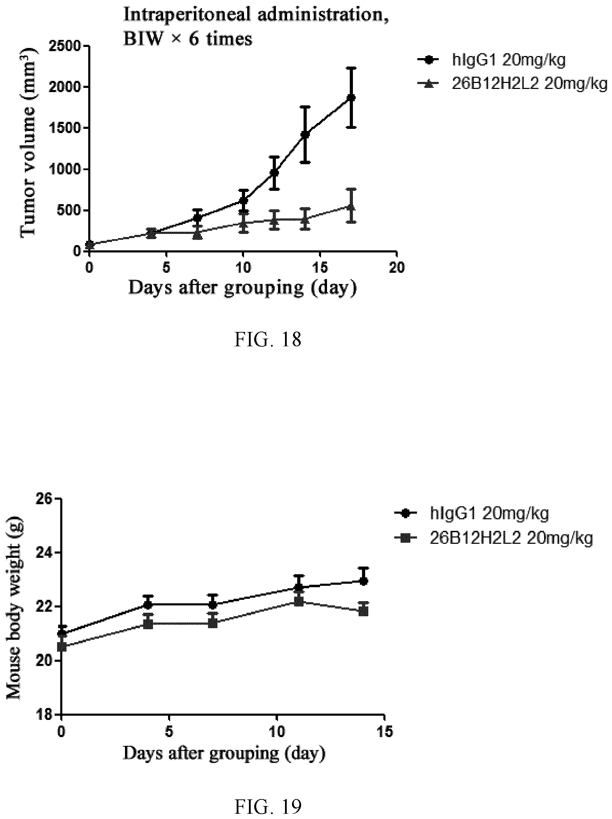

The experimental results are shown in FIG. 18.

The results show that 26B12H2L2 caused significant reductions in tumor volume in the hTIGIT-BALB/c transgenic mouse CT26 tumor model.

The results demonstrate that 26B12H2L2 had potent efficacy on the hTIGIT-BALB/c transgenic mouse CT26 tumor model, thus having the potential to treat and/or prevent tumors, particularly colon cancer.

Meanwhile, as shown in FIG. 19, 26B12H2L2 had no effect on the body weight of the hTIGIT-BALB/c transgenic mice as the CT26 tumor model, indicating that the antibody 26B12H2L2 did not cause toxic side effects on the mice.

### Example 11: Effective Treatment of Anti-TIGIT Antibody in Combination with Anti-CTLA4-Anti-PD-1 Bispecific Antibody

In order to determine the anti-tumor activity *in vivo* of the anti-TIGIT antibody in combination with the anti-CTLA4-anti-PD-1 bispecific antibody CP004(hG1TM), CT26 cells (human colon cancer cells, purchased from GemPharmatech Co., Ltd.) were first inoculated subcutaneously into female BALB/c-hPD1/hTIGIT mice aged 5-7 weeks (purchased from GemPharmatech Co., Ltd.), and when the mean tumor volume reached 80-120 mm³, the mice were randomly divided into 4 groups of 6 mice per group based on tumor volume. The day of grouping was defined as D0, and administration was started on the day of grouping D0. The regimen of the combination therapy group was as follows: the drugs were formulated separately and administered sequentially (no certain order or time interval was required, and one treatment should be given after the completed administration of the other). The modeling and specific regimen are shown in Table 10. After the administration, the length and width of tumors in each group were measured, and the tumor volume was calculated.

**Table 10: Dose regimen of anti-TIGIT antibody in combination with anti-CTLA4-anti-PD-1 bispecific antibody for treating CT26 graft tumor in BALB/c-hPD1/hTIGIT mouse model**

| Grouping | n | Tumor xenograft | Condition of administration |
|---|---|---|---|
| Isotype control | 6 | CT26, 5 × 10⁵ cells, BALB/c-hPD1/ hTIGIT mice, SC | Isotype control antibody hIgG, 5 mg/kg; Intraperitoneally administered twice weekly for 4 weeks |
| 5 mg/kg | | | |
| CP004(hG1TM) | 6 | | CP004(hG1TM), 0.5 mg/kg, Intraperitoneally administered twice weekly for 4 weeks |
| 0.5mg/kg | | | |
| 26B12H2L2 | 6 | | 26B 12H2L2, 4 mg/kg; Intraperitoneally administered twice weekly for 4 weeks |
| 4mg/kg | | | |
| 26B 12H2L2 | 6 | | 26B12H2L2, 4 mg/kg;CP004(hG1TM), 0.5 mg/kg; |
| 4mg/kg, | | | |
| CP004(hG 1 TM) | | | Intraperitoneally administered twice weekly for 4 weeks |
| 0.5mg/kg | | | |

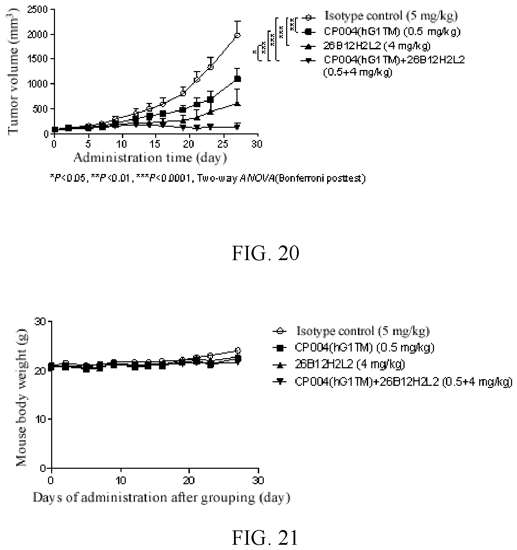

The results are shown in FIG. 20. The results show that: compared with the isotype control antibody hIgG, both CP004(hG1TM) and 26B12H2L2 could effectively inhibit the growth of mouse tumors. The CP004(hG1TM) + 26B12H2L2 group exhibited synergistic anti-tumor effects on the model, and the combination therapy showed anti-tumor inhibition superior to that of the monotherapies.

In addition, as shown in FIG. 21, both CP004(hG1TM) and 26B12H2L2 were well tolerated by the tumor-bearing mice, either alone or in combination, and no effect on the body weight of the tumor-bearing mice was found in the groups.

Although specific embodiments of the present invention have been described in detail, those skilled in the art will appreciate that various modifications and substitutions can be made to those details according to all the teachings that have been disclosed, and these changes shall all fall within the protection scope of the present invention. The full scope of the present invention is given by the appended claims and any equivalent thereof.

### SEQUENCE LISTING (Note: those underlined are CDR sequences)

Amino acid sequence of 26B12VH 
EVQLQESGPGLVKPSQSLSLTCTVTGHSFTSDYAWNWIRQFPGNRLEWMGYISYSDSTN
YNPSLKSRISITRDTSKNQFFLQMNSVTTEDTATYYCARLDYGNYGGAMDYWGQGTSV
TVSS (SEQ ID NO: 1)
Nucleotide sequence of 26B 12VH 
GAGGTGCAGCTGCAGGAGTCTGGACCTGGCCTGGTGAAACCCTCTCAGTCTCTGTCC
CTCACCTGCACTGTCACTGGCCACTCATTCACCAGTGATTATGCCTGGAACTGGATCC
GGCAGTTTCCAGGAAACAGACTGGAGTGGATGGGCTACATAAGCTACAGTGATAGC
ACTAACTACAACCCATCTCTCAAAAGTCGAATCTCTATCACTCGAGACACATCCAAG
AACCAGTTCTTCTTGCAGATGAATTCTGTGACTACTGAGGACACAGCCACATATTAC
TGTGCAAGATTGGACTATGGTAACTACGGTGGGGCTATGGACTACTGGGGTCAAGGG
ACCTCAGTCACCGTCTCCTCA (SEQ ID NO: 2)
HCDR1: GHSFTSDYA (SEQ ID NO: 3)
HCDR2: ISYSDST (SEQ ID NO: 4)
HCDR3: ARLDYGNYGGAMDY (SEQ ID NO: 5)
Amino acid sequence of 26B12VL 
DIVLTQSHEFMSTSLRDRVSITCKSSQHVSTAVAWYQQKPGQSPKLLIYSASYRYTGVPD
RFTGSGSGTDFTFTISSVKAEDLAVYYCQQHYITPWTFGGGTKLEIK (SEQ ID NO: 6)
Nucleotide sequence of 26B 12VL 
GATATTGTGCTAACTCAGTCTCACGAATTCATGTCCACCTCATTACGAGACAGGGTC
AGCATCACCTGCAAATCCAGTCAACATGTGAGTACTGCTGTAGCCTGGTATCAACAG
AAACCAGGACAATCTCCTAAACTACTGATTTACTCGGCATCCTACCGGTACACTGGA
GTCCCTGATCGCTTCACTGGCAGTGGATCTGGGACGGATTTCACTTTCACCATCAGCA
GTGTGAAGGCTGAAGACCTGGCAGTTTATTACTGTCAGCAACATTATATTACTCCGT
GGACGTTCGGTGGAGGCACCAAGCTGGAAATAAAA (SEQ ID NO: 7)
LCDR1: QHVSTA (SEQ ID NO: 8)
LCDR2: SAS (SEQ ID NO: 9)
LCDR3: QQHYITPWT (SEQ ID NO: 10)
Amino acid sequence of 26B12H1 
DVQLQESGPGLVKPSQTLSLTCTVSGHSFTSDYAWNWIRQFPGKGLEWIGYISYSDSTNY
NPSLKSRITISRDTSKNQFFLQLNSVTAADTATYYCARLDYGNYGGAMDYWGQGTSVT
VSS (SEQ ID NO: 11)
Nucleotide sequence of 26B 12H 1 
GATGTGCAGCTGCAGGAGAGCGGCCCCGGACTGGTGAAGCCTTCCCAGACCCTGTCT
CTGACCTGTACAGTGTCTGGCCACAGCTTCACATCCGACTACGCCTGGAACTGGATC
AGGCAGTTTCCAGGCAAGGGCCTGGAGTGGATCGGCTACATCTCTTATAGCGACTCC
ACCAACTATAATCCCTCTCTGAAGAGCCGGATCACCATCAGCAGAGATACATCCAAG
AACCAGTTCTTTCTGCAGCTGAACAGCGTGACAGCCGCCGACACCGCCACATACTAT
TGCGCCCGGCTGGACTACGGCAATTATGGCGGAGCCATGGATTACTGGGGCCAGGG
CACCTCCGTGACAGTGAGCTCC (SEQ ID NO: 12)
Amino acid sequence of 26B12H2 
DVQLQESGPGLVKPSQTLSLTCTVSGHSFTSDYAWSWIRQPPGKGLEWIGYISYSDSTNY
NPSLKSRVTISRDTSKNQFSLKLSSVTAADTAVYYCARLDYGNYGGAMDYWGQGTSVT
VSS (SEQ ID NO: 13)
Nucleotide sequence of 26B 12H2 
GATGTGCAGCTGCAGGAGTCTGGCCCAGGACTGGTGAAGCCAAGCCAGACCCTGTC
CCTGACCTGTACAGTGTCCGGCCACTCTTTTACAAGCGACTACGCCTGGTCTTGGATC
AGGCAGCCCCCTGGCAAGGGACTGGAGTGGATCGGCTACATCTCCTATTCTGACAGC
ACCAACTATAATCCCTCCCTGAAGTCTCGGGTGACCATCTCTAGAGATACAAGCAAG
AACCAGTTCTCCCTGAAGCTGAGCTCCGTGACCGCAGCAGACACAGCCGTGTACTAT
TGCGCCCGGCTGGACTACGGCAATTATGGCGGAGCCATGGATTACTGGGGCCAGGG
CACCAGCGTGACAGTGTCTAGC (SEQ ID NO: 14)
Amino acid sequence of 26B12H3 
DVQLQESGPGLVKPSQTLSLTCTVSGHSFTSDYAWSWIRQPPGKGLEWIGYISYSDSTNY
NPSLKSRVTISVDTSKNQFSLKLSSVTAADTAVYYCARLDYGNYGGAMDYWGQGTSVT
VSS (SEQ ID NO: 15)
Nucleotide sequence of 26B 12H3 
GATGTGCAGCTGCAGGAGTCTGGCCCAGGACTGGTGAAGCCAAGCCAGACCCTGTC
CCTGACCTGTACAGTGTCCGGCCACTCTTTTACAAGCGACTACGCCTGGTCTTGGATC
AGACAGCCCCCTGGCAAGGGACTGGAGTGGATCGGCTACATCTCCTATTCTGACAGC
ACCAACTATAATCCCTCCCTGAAGTCTAGAGTGACCATCTCTGTGGATACAAGCAAG
AACCAGTTCTCCCTGAAGCTGAGCTCCGTGACCGCAGCAGACACAGCCGTGTACTAT
TGCGCCCGGCTGGACTACGGCAATTATGGCGGAGCCATGGATTACTGGGGCCAGGG
CACCAGCGTGACAGTGTCTAGC (SEQ ID NO: 16)
Amino acid sequence of 26B12H4 
DVQLQESGPGLVKPSQTLSLTCTVSGHSFTSDYAWNWIRQFPGKGLEWMGYISYSDSTN
YNPSLKSRITISRDTSKNQFFLQLNSVTAADTATYYCARLDYGNYGGAMDYWGQGTSV
TVSS (SEQ ID NO: 17)
Nucleotide sequence of 26B 12H4 
GATGTGCAGCTGCAGGAGAGCGGCCCCGGACTGGTGAAGCCTTCCCAGACCCTGTCT
CTGACCTGTACAGTGTCTGGCCACAGCTTCACATCCGACTACGCCTGGAACTGGATC
AGGCAGTTTCCAGGCAAGGGCCTGGAGTGGATGGGCTACATCTCTTATAGCGACTCC
ACCAACTATAATCCCTCTCTGAAGAGCCGGATCACCATCAGCAGAGATACATCCAAG
AACCAGTTCTTTCTGCAGCTGAACAGCGTGACAGCCGCCGACACCGCCACATACTAT
TGCGCCCGGCTGGACTACGGCAATTATGGCGGAGCCATGGATTACTGGGGCCAGGG
CACCTCCGTGACAGTGAGCTCC (SEQ ID NO: 18)
Amino acid sequence of 26B12L1 
DIQMTQSPKSLSTSVGDRVTITCRSSQHVSTAVAWYQQKPGKSPKLLIYSASYRYSGVPD
RFSGSGSGTDFTFTISSVQPEDFATYYCQQHYITPWTFGGGTKLEIK (SEQ ID NO: 19)
Nucleotide sequence of 26B 12L1 
GACATCCAGATGACCCAGTCCCCTAAGTCCCTGTCTACAAGCGTGGGCGATCGGGTG
ACCATCACATGTAGAAGCTCCCAGCACGTGTCTACCGCAGTGGCATGGTACCAGCAG
AAGCCAGGCAAGAGCCCTAAGCTGCTGATCTATTCCGCCTCTTACAGGTATTCCGGA
GTGCCAGACCGGTTTAGCGGCTCCGGCTCTGGCACCGATTTCACCTTTACAATCTCTA
GCGTGCAGCCAGAGGACTTCGCCACATACTATTGCCAGCAGCACTACATCACCCCAT
GGACCTTCGGCGGCGGCACAAAGCTGGAGATCAAG (SEQ ID NO: 20)
Amino acid sequence of 26B12L2 
DIQMTQSPSSLSASVGDRVTITCRSSQHVSTALAWYQQKPGKSPKLLIYSASSRYSGVPD
RFSGSGSGTDFTFTISSLQPEDFATYYCQQHYITPWTFGGGTKLEIK (SEQ ID NO: 21)
Nucleotide sequence of 26B 12L2 
GACATCCAGATGACCCAGTCCCCTAGCTCCCTGTCTGCCAGCGTGGGCGATAGGGTG
ACCATCACATGTAGATCTAGCCAGCACGTGTCTACAGCCCTGGCATGGTACCAGCAG
AAGCCAGGCAAGAGCCCTAAGCTGCTGATCTACTCCGCCTCCTCTAGGTATTCTGGA
GTGCCAGACCGGTTTTCCGGCTCTGGCAGCGGCACCGATTTCACCTTTACAATCAGCT
CCCTGCAGCCAGAGGACTTCGCCACATACTATTGCCAGCAGCACTATATCACCCCAT
GGACCTTCGGCGGCGGCACCAAGCTGGAGATCAAG (SEQ ID NO: 22)
Amino acid sequence of 26B12L3 
DIQMTQSPSSLSASVGDRVTITCRASQHVSTALAWYQQKPGKAPKLLIYSASSLQSGVPS
RFSGSGSGTDFTLTISSLQPEDFATYYCQQHYITPWTFGGGTKLEIK (SEQ ID NO: 23)
Nucleotide sequence of 26B 12L3 
GACATCCAGATGACCCAGTCCCCTAGCTCCCTGAGCGCCTCCGTGGGCGATAGGGTG
ACCATCACATGTAGAGCCTCTCAGCACGTGAGCACAGCCCTGGCATGGTACCAGCAG
AAGCCAGGCAAGGCCCCTAAGCTGCTGATCTATAGCGCCTCTAGCCTGCAGTCCGGA
GTGCCATCTCGGTTCTCTGGCAGCGGCTCCGGAACCGACTTTACCCTGACAATCTCCT
CTCTGCAGCCAGAGGATTTCGCCACATACTATTGCCAGCAGCACTACATCACCCCAT
GGACCTTCGGCGGCGGCACCAAGCTGGAGATCAAG (SEQ ID NO: 24)
Amino acid sequence of 26B12L4 
DIQMTQSPKSMSTSVGDRVTITCRSSQHVSTAVAWYQQKPGKSPKLLIYSASYRYSGVP
DRFSGSGSGTDFTFTISSVQPEDFATYYCQQHYITPWTFGGGTKLEIK (SEQ ID NO: 25)
Nucleotide sequence of 26B 12L4

GACATCCAGATGACCCAGTCCCCTAAGTCCATGTCTACAAGCGTGGGCGACAGGGTG
ACCATCACATGTAGAAGCTCCCAGCACGTGTCTACCGCAGTGGCATGGTACCAGCAG
AAGCCAGGCAAGAGCCCTAAGCTGCTGATCTATTCCGCCTCTTACAGGTATTCCGGA
GTGCCAGACCGGTTTAGCGGCTCCGGCTCTGGCACCGATTTCACCTTTACAATCTCTA
GCGTGCAGCCAGAGGACTTCGCCACATACTATTGCCAGCAGCACTACATCACCCCAT
GGACCTTCGGCGGCGGCACAAAGCTGGAGATCAAG (SEQ ID NO: 26)

The following is the sequence of CTLA4-PD1
Amino acid sequence of 14C12 heavy chain variable region 
EVKLVESGGGLVKPGGSLKLSCAASGFAFSSYDMSWVRQTPEKRLEWVATISGGGRYT
YYPDSVKGRFTISRDNARNTLYLQMSSLRSEDTALYYCANRYGEAWFAYWGQGTLVTV
SA (SEQ ID NO: 27)
Amino acid sequence of 14C12 light chain variable region 
DIKMTQSPSSMYASLGERVTFTCKASQDINTYLSWFQQKPGKSPKTLIYRANRLVDGVPS
RFSGSGSGQDYSLTISSLEYEDMGIYYCLQYDEFPLTFGAGTKLELK (SEQ ID NO: 28)
HCDR1 of 14C12: Gly Phe Ala Phe Ser Ser Tyr Asp (SEQ ID NO: 29)
HCDR2 of 14C12: Ile Ser Gly Gly Gly Arg Tyr Thr (SEQ ID NO: 30)
HCDR3 of 14C12: Ala Asn Arg Tyr Gly Glu Ala Trp Phe Ala Tyr (SEQ ID NO: 31)
LCDR1 of 14C12: Gin Asp Ile Asn Thr Tyr (SEQ ID NO: 32)
LCDR2 of 14C12: Arg Ala Asn (SEQ ID NO: 33)
LCDR3 of 14C12: Leu Gln Tyr Asp Glu Phe Pro Leu Thr (SEQ ID NO: 34)
Amino acid sequence of 4G10 heavy chain variable region 
QVKLQESGPELVKPGASMKISCKASGYSFTGYTMNWVKQSHGKNLEWIGLINPYNNITN
YNQKFMGKATFTVDKSSSTAYMELLRLTSEDSGVYFCARLDYRSYWGQGTLVTVSAAK
TTPPSVY (SEQ ID NO: 35)
Amino acid sequence of 4G10 light chain variable region 
QAVVTQESALTTSPGETVTLTCRSSTGAVTTSNFANWVQEKPDHLFTSLIGGTNNRAPGV
PARFSGSLIGDKAALTITGAQTEDEAIYFCALWYSNHWVFGGGTKLTVLGQPKSSPSVTL
FQGQFC (SEQ ID NO: 36)
HCDR1 of 4G10: Gly Tyr Ser Phe Thr Gly Tyr Thr (SEQ ID NO: 37)
HCDR2 of 4G10: Ile Asn Pro Tyr Asn Asn Ile Thr (SEQ ID NO: 38)
HCDR3 of 4G10: Ala Arg Leu Asp Tyr Arg Ser Tyr (SEQ ID NO: 39)
LCDR1 of 4G10: Thr Gly Ala Val Thr Thr Ser Asn Phe (SEQ ID NO: 40)
LCDR2 of 4G10: Gly Thr Asn (SEQ ID NO: 41)
LCDR3 of 4G10: Ala Leu Trp Tyr Ser Asn His Trp Val (SEQ ID NO: 42)
Amino acid sequence of 14C12H1L1 heavy chain variable region 
EVQLVESGGGLVQPGGSLRLSCAASGFAFSSYDMSWVRQAPGKGLDWVATISGGGRYT
YYPDSVKGRFTISRDNSKNNLYLQMNSLRAEDTALYYCANRYGEAWFAYWGQGTLVT
VSS (SEQ ID NO: 43)
Amino acid sequence of 14C12H1L1 light chain variable region 
DIQMTQSPSSMSASVGDRVTFTCRASQDINTYLSWFQQKPGKSPKTLIYRANRLVSGVPS
RFSGSGSGQDYTLTISSLQPEDMATYYCLQYDEFPLTFGAGTKLELK (SEQ ID NO: 44)
Amino acid sequence of 4G10H1L1 heavy chain variable region 
QVQLVESGAELVKPGASMKISCKASGYSFTGYTMNWVKQAPGQGLEWIGLINPYNNITN
YNQKFMGKATFTVDKSISTAYMELSRLTSDDSGVYFCARLDYRSYWGQGTLVTVSA
(SEQ ID NO: 45)
Amino acid sequence of 4G10H3L3 heavy chain variable region 
QVQLVESGAEVKKPGASVKVSCKASGYSFTGYTMNWVRQAPGQGLEWIGLINPYNNIT
NYAQKFQGRVTFTVDTSISTAYMELSRLRSDDTGVYFCARLDYRSYWGQGTLVTVSA
(SEQ ID NO: 46)
Amino acid sequence of 4G10H1V(M) 
QVQLVESGAELVKPGASMKISCKASGYSFTGYTMNWVKQAPGQCLEWIGLINPYNNITN
YNQKFMGKATFTVDKSISTAYMELSRLTSDDSGVYFCARLDYRSYWGQGTLVTVSA
(SEQ ID NO: 47)
Amino acid sequence of 4G10H3V(M) 
QVQLVESGAEVKKPGASVKVSCKASGYSFTGYTMNWVRQAPGQCLEWIGLINPYNNIT
NYAQKFQGRVTFTVDTSISTAYMELSRLRSDDTGVYFCARLDYRSYWGQGTLVTVSA
(SEQ ID NO: 48)
Amino acid sequence of 4G10H1L1 light chain variable region 
QAVVTQEPSLTVSPGGTVTLTCGSSTGAVTTSNFANWVQEKPGQAFRSLIGGTNNRASW
VPARFSGSLLGGKAALTISGAQPEDEAEYFCALWYSNHWVFGGGTKLTVL (SEQ ID NO:
 49)
Amino acid sequence of 4G10H3L3 light chain variable region 
QAVVTQEPSLTVSPGGTVTLTCGSSTGAVTTSNFPNWVQQKPGQAPRSLIGGTNNKASW
TPARFSGSLLGGKAALTISGAQPEDEAEYYCALWYSNHWVFGGGTKLTVL (SEQ ID NO:
50)
Amino acid sequence of 4G10L1V(M) 
QAVVTQEPSLTVSPGGTVTLTCGSSTGAVTTSNFANWVQEKPGQAFRSLIGGTNNRASW
VPARFSGSLLGGKAALTISGAQPEDEAEYFCALWYSNHWVFGCGTKLTVLR (SEQ ID
NO: 51)
Amino acid sequence of 4G10L3V(M) 
QAVVTQEPSLTVSPGGTVTLTCGSSTGAVTTSNFPNWVQQKPGQAPRSLIGGTNNKASW
TPARFSGSLLGGKAALTISGAQPEDEAEYYCALWYSNHWVFGCGTKLTVLR (SEQ ID
NO: 52)
Amino acid sequence of the heavy chain of the immunoglobulin moiety in CP004(hG1TM) 
EVQLVESGGGLVQPGGSLRLSCAASGFAFSSYDMSWVRQAPGKGLDWVATISGGGRYT
YYPDSVKGRFTISRDNSKNNLYLQMNSLRAEDTALYYCANRYGEAWFAYWGQGTLVT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEA
AGAPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLP
PSRDELTKNQVSLTCLVKGFYPSDIAVEWESNGQPENNYKTTPPVLDSDGSFFLYSKLTV
DKSRWQQGNVFSCSVMHEALHNHYTQKSLSLSPGK (SEQ ID NO: 53)
Encoded amino acid sequence of 14C12H1L1 light chain (14C12L1): (214 aa) 
DIQMTQSPSSMSASVGDRVTFTCRASQDINTYLSWFQQKPGKSPKTLIYRANRLVSGVPS
RFSGSGSGQDYTLTISSLQPEDMATYYCLQYDEFPLTFGAGTKLELKRTVAAPSVFIFPPS
DEQLKSGTASVVCLLNNFYPREAKVQWKVDNALQSGNSQESVTEQDSKDSTYSLSSTLT
LSKADYEKHKVYACEVTHQGLSSPVTKSFNRGEC (SEQ ID NO: 54)
Amino acid sequence of Linker1: GGGGSGGGGSGGGGS (SEQ ID NO: 55)
Amino acid sequence of Linker2: GGGGSGGGGSGGGGSGGGGS (SEQ ID NO: 56)
Amino acid sequence of CP004(hG 1 TM) heavy chain 
EVQLVESGGGLVQPGGSLRLSCAASGFAFSSYDMSWVRQAPGKGLDWVATISGGGRYT
YYPDSVKGRFTISRDNSKNNLYLQMNSLRAEDTALYYCANRYGEAWFAYWGQGTLVT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEA
AGAPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLP
PSRDELTKNQVSLTCLVKGFYPSDIAVEWESNGQPENNYKTTPPVLDSDGSFFLYSKLTV
DKSRWQQGNVFSCSVMHEALHNHYTQKSLSLSPGKGGGGSGGGGSGGGGSGGGGSQV
QLVESGAEVKKPGASVKVSCKASGYSFTGYTMNWVRQAPGQCLEWIGLINPYNNITNY
AQKFQGRVTFTVDTSISTAYMELSRLRSDDTGVYFCARLDYRSYWGQGTLVTVSAGGG
GSGGGGSGGGGSGGGGSQAVVTQEPSLTVSPGGTVTLTCGSSTGAVTTSNFPNWVQQK
PGQAPRSLIGGTNNKASWTPARFSGSLLGGKAALTISGAQPEDEAEYYCALWYSNHWVF
GCGTKLTVLR (SEQ ID NO: 57)
Nucleotide sequence of CP004(hG 1 TM) heavy chain 
GAAGTGCAGCTGGTCGAGTCTGGGGGAGGGCTGGTGCAGCCCGGCGGGTCACTGCG
ACTGAGCTGCGCAGCTTCCGGATTCGCCTTTAGCTCCTACGACATGTCCTGGGTGCG
 
ACAGGCACCAGGAAAGGGACTGGATTGGGTCGCTACTATCTCAGGAGGCGGGAGAT
ACACCTACTATCCTGACAGCGTCAAGGGCCGGTTCACAATCTCTAGAGATAACAGTA
AGAACAATCTGTATCTGCAGATGAACAGCCTGAGGGCTGAGGACACCGCACTGTACT
ATTGTGCCAACCGCTACGGGGAAGCATGGTTTGCCTATTGGGGGCAGGGAACCCTGG
TGACAGTCTCTAGTGCCAGCACCAAAGGGCCCAGCGTGTTTCCTCTCGCCCCCTCCTC
CAAAAGCACCAGCGGAGGAACCGCTGCTCTCGGATGTCTGGTGAAGGACTACTTCCC
TGAACCCGTCACCGTGAGCTGGAATAGCGGCGCTCTGACAAGCGGAGTCCATACATT
CCCTGCTGTGCTGCAAAGCAGCGGACTCTATTCCCTGTCCAGCGTCGTCACAGTGCC
CAGCAGCAGCCTGGGCACCCAGACCTACATCTGTAACGTCAACCACAAGCCCTCCAA
CACCAAGGTGGACAAGAAAGTGGAGCCCAAATCCTGCGACAAGACACACACCTGTC
CCCCCTGTCCTGCTCCCGAAGCTGCTGGAGCCCCTAGCGTCTTCCTCTTTCCTCCCAA
ACCCAAGGACACCCTCATGATCAGCAGAACCCCTGAAGTCACCTGTGTCGTCGTGGA
TGTCAGCCATGAGGACCCCGAGGTGAAATTCAACTGGTATGTCGATGGCGTCGAGGT
GCACAACGCCAAAACCAAGCCCAGGGAGGAACAGTACAACTCCACCTACAGGGTGG
TGTCCGTGCTGACAGTCCTCCACCAGGACTGGCTGAACGGCAAGGAGTACAAGTGCA
AGGTGTCCAACAAGGCTCTCCCTGCCCCCATTGAGAAGACCATCAGCAAGGCCAAA
GGCCAACCCAGGGAGCCCCAGGTCTATACACTGCCTCCCTCCAGGGACGAACTCACC
AAGAACCAGGTGTCCCTGACCTGCCTGGTCAAGGGCTTTTATCCCAGCGACATCGCC
GTCGAGTGGGAGTCCAACGGACAGCCCGAGAATAACTACAAGACCACCCCTCCTGT
CCTCGACTCCGACGGCTCCTTCTTCCTGTACAGCAAACTGACCGTGGATAAGTCCAG
ATGGCAGCAGGGCAATGTCTTTTCATGTTCCGTGATGCACGAGGCACTGCACAACCA
CTATACCCAGAAGTCTCTGAGTCTGTCACCAGGAAAAGGAGGAGGAGGCTCTGGAG
GAGGCGGAAGTGGAGGCGGAGGATCAGGAGGGGGAGGATCTCAGGTGCAGCTGGT
CGAATCCGGAGCCGAGGTGAAGAAACCCGGCGCTTCCGTGAAGGTCTCTTGCAAAG
CATCAGGCTACAGCTTCACAGGGTATACTATGAACTGGGTGCGGCAGGCACCTGGAC
AGTGTCTGGAATGGATCGGCCTGATTAACCCATACAACAACATCACTAACTACGCCC
AGAAGTTCCAGGGCCGGGTGACTTTTACCGTGGACACTAGCATTTCCACCGCTTACA
TGGAGCTGAGTCGGCTGAGATCAGACGATACCGGCGTGTATTTTTGCGCAAGGCTGG
 
ATTACAGAAGTTATTGGGGACAGGGAACACTTGTTACAGTCTCTGCTGGAGGAGGCG
GATCTGGAGGAGGAGGATCTGGCGGAGGAGGCAGTGGAGGAGGAGGATCACAGGC
TGTGGTTACTCAGGAACCAAGCCTGACCGTGAGCCCCGGAGGCACAGTCACTCTGAC
CTGTGGGAGCTCCACAGGAGCTGTGACCACATCTAACTTCCCTAATTGGGTGCAGCA
GAAGCCAGGACAGGCACCTCGATCCCTGATCGGGGGAACCAACAACAAGGCCAGCT
GGACACCCGCCAGATTTTCTGGCAGTCTGCTGGGCGGGAAAGCCGCTCTGACCATTA
GCGGCGCTCAGCCTGAGGACGAAGCAGAGTACTATTGCGCCCTGTGGTATAGTAATC
ATTGGGTGTTCGGGTGTGGGACAAAACTGACCGTGCTGAGA (SEQ ID NO: 58)
Amino acid sequence of CP004(hG 1 TM) light chain 
DIQMTQSPSSMSASVGDRVTFTCRASQDINTYLSWFQQKPGKSPKTLIYRANRLVSGVPS
RFSGSGSGQDYTLTISSLQPEDMATYYCLQYDEFPLTFGAGTKLELKRTVAAPSVFIFPPS
DEQLKSGTASVVCLLNNFYPREAKVQWKVDNALQSGNSQESVTEQDSKDSTYSLSSTLT
LSKADYEKHKVYACEVTHQGLSSPVTKSFNRGEC (SEQ ID NO: 59)
Nucleotide sequence of CP004(hG 1 TM) light chain 
GACATTCAGATGACTCAGAGCCCCTCCTCCATGTCCGCCTCTGTGGGCGACAGGGTC
ACCTTCACATGCCGCGCTAGTCAGGATATCAACACCTACCTGAGCTGGTTTCAGCAG
AAGCCAGGGAAAAGCCCCAAGACACTGATCTACCGGGCTAATAGACTGGTGTCTGG
AGTCCCAAGTCGGTTCAGTGGCTCAGGGAGCGGACAGGACTACACTCTGACCATCAG
CTCCCTGCAGCCTGAGGACATGGCAACCTACTATTGCCTGCAGTATGATGAGTTCCC
ACTGACCTTTGGCGCCGGGACAAAACTGGAGCTGAAGCGAACTGTGGCCGCTCCCTC
CGTCTTCATTTTTCCCCCTTCTGACGAACAGCTGAAATCAGGCACAGCCAGCGTGGTC
TGTCTGCTGAACAATTTCTACCCTAGAGAGGCAAAAGTGCAGTGGAAGGTCGATAAC
GCCCTGCAGTCCGGCAACAGCCAGGAGAGTGTGACTGAACAGGACTCAAAAGATAG
CACCTATTCCCTGTCTAGTACACTGACTCTGTCCAAGGCTGATTACGAGAAGCACAA
AGTGTATGCATGCGAAGTGACACATCAGGGACTGTCAAGCCCCGTGACTAAGTCTTT
TAACCGGGGCGAATGT (SEQ ID NO: 60)
Amino acid sequence of CP004(hG 1 TM) heavy chain CDR
   HCDR1: GFAFSSYD (SEQ ID NO: 61)
   HCDR2: ISGGGRYT (SEQ ID NO: 62)
   HCDR3: ANRYGEAWFAY (SEQ ID NO: 63)
   HCDR4: GYSFTGYT (SEQ ID NO: 64)
   HCDR5: INPYNNIT (SEQ ID NO: 65)
   HCDR6: ARLDYRSY (SEQ ID NO: 66)
   HCDR7: TGAVTTSNF (SEQ ID NO: 67)
   HCDR8: GTN (SEQ ID NO: 68)
   HCDR9: ALWYSNHWV (SEQ ID NO: 69)
Amino acid sequence of CP004(hG 1 TM) light chain CDR
   LCDR1: QDINTY (SEQ ID NO: 70)
   LCDR2: RAN (SEQ ID NO: 71)
   LCDR3: LQYDEFPLT (SEQ ID NO: 72)
Amino acid sequence of CP004(hG 1 TM) heavy chain variable region
   VH1: 
EVQLVESGGGLVQPGGSLRLSCAASGFAFSSYDMSWVRQAPGKGLDWVATISGGGRYT
YYPDSVKGRFTISRDNSKNNLYLQMNSLRAEDTALYYCANRYGEAWFAYWGQGTLVT
VSS (SEQ ID NO: 73)
   VH2: 
QVQLVESGAEVKKPGASVKVSCKASGYSFTGYTMNWVRQAPGQCLEWIGLINPYNNIT
NYAQKFQGRVTFTVDTSISTAYMELSRLRSDDTGVYFCARLDYRSYWGQGTLVTVSA
(SEQ ID NO: 74)
   VH3: 
QAVVTQEPSLTVSPGGTVTLTCGSSTGAVTTSNFPNWVQQKPGQAPRSLIGGTNNKASW
TPARFSGSLLGGKAALTISGAQPEDEAEYYCALWYSNHWVFGCGTKLTVLR (SEQ ID
NO: 75)
Amino acid sequence of CP004(hG 1 TM) light chain variable region 
DIQMTQSPSSMSASVGDRVTFTCRASQDINTYLSWFQQKPGKSPKTLIYRANRLVSGVPS
RFSGSGSGQDYTLTISSLQPEDMATYYCLQYDEFPLTFGAGTKLELK (SEQ ID NO: 76)
Amino acid sequence of mFc: 
PRGPTIKPCPPCKCPAPNLLGGPSVFIFPPKIKDVLMISLSPIVTCVVVDVSEDDPDVQISWF
VNNVEVHTAQTQTHREDYNSTLRVVSALPIQHQDWMSGKEFKCKVNNKDLPAPIERTIS
KPKGSVRAPQVYVLPPPEEEMTKKQVTLTCMVTDFMPEDIYVEWTNNGKTELNYKNTE
PVLDSDGSYFMYSKLRVEKKNWVERNSYSCSVVHEGLHNHHTTKSFSRTPGK (SEQ ID
NO: 77)
hFc sequence

THTCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKAK
GQPREPQVYTLPPSRDELTKNQVSLTCLVKGFYPSDIAVEWESNGQPENNYKTTPPVLDS
DGSFFLYSKLTVDKSRWQQGNVFSCSVMHEALHNHYTQKSLSLSPGK (SEQ ID NO: 78)

## Claims

1. A pharmaceutical composition or a kit comprising an anti-TIGIT antibody or an antigen-binding fragment thereof, and an anti-CTLA4-anti-PD-1 bispecific antibody or an antigen-binding fragment thereof, wherein optionally, the pharmaceutical composition further comprises a pharmaceutically acceptable carrier and/or excipient,
wherein the anti-TIGIT antibody comprises HCDR1-HCDR3 contained in a heavy chain variable region set forth in SEQ ID NO: 1 and LCDR1-LCDR3 contained in a light chain variable region set forth in SEQ ID NO: 6 (preferably, according to the IMGT numbering system, the antibody comprises a heavy chain variable region comprising HCDR1-HCDR3 having amino acid sequences set forth in SEQ ID NOs: 3-5, respectively, and a light chain variable region comprising LCDR1-LCDR3 having amino acid sequences set forth in SEQ ID NOs: 8-10, respectively),
and the anti-CTLA4-anti-PD-1 bispecific antibody comprises:
a first protein functional region targeting PD-1 and
a second protein functional region targeting CTLA4,
wherein the first protein functional region is an immunoglobulin, and the second protein functional region is a single chain antibody, or the first protein functional region is a single chain antibody, and the second protein functional region is an immunoglobulin,
wherein
the immunoglobulin comprises HCDR1-HCDR3 (preferably HCDR1-HCDR3 set forth in SEQ ID NOs: 29-31, respectively) in a heavy chain variable region set forth in SEQ ID NO: 27 and LCDR1-LCDR3 (preferably LCDR1-LCDR3 set forth in SEQ ID NOs: 32-34, respectively) in a light chain variable region set forth in SEQ ID NO: 28; the single chain antibody comprises HCDR1-HCDR3 (preferably HCDR1-HCDR3 set forth in SEQ ID NOs: 37-39, respectively) in a heavy chain variable region set forth in SEQ ID NO: 35 and LCDR1-LCDR3 (preferably LCDR1-LCDR3 set forth in SEQ ID NOs: 40-42, respectively) in a light chain variable region set forth in SEQ ID NO: 36;
or
the immunoglobulin comprises HCDR1-HCDR3 (preferably HCDR1-HCDR3 set forth in SEQ ID NOs: 37-39, respectively) in a heavy chain variable region set forth in SEQ ID NO: 35 and LCDR1-LCDR3 (preferably LCDR1-LCDR3 set forth in SEQ ID NOs: 40-42, respectively) in a light chain variable region set forth in SEQ ID NO: 36; the single chain antibody comprises HCDR1-HCDR3 (preferably HCDR1-HCDR3 set forth in SEQ ID NOs: 29-31, respectively) in a heavy chain variable region set forth in SEQ ID NO: 27 and LCDR1-LCDR3 (preferably LCDR1-LCDR3 set forth in SEQ ID NOs: 32-34, respectively) in a light chain variable region set forth in SEQ ID NO: 28.

2. The pharmaceutical composition or the kit according to claim 1, wherein an amino acid sequence of the heavy chain variable region of the anti-TIGIT antibody is selected from SEQ ID NO: 1, SEQ ID NO: 11, SEQ ID NO: 13, SEQ ID NO: 15, and SEQ ID NO: 17, and an amino acid sequence of the light chain variable region of the anti-TIGIT antibody is selected from SEQ ID NO: 6, SEQ ID NO: 19, SEQ ID NO: 21, SEQ ID NO: 23, and SEQ ID NO: 25;
preferably, for the anti-TIGIT antibody or the antigen-binding fragment thereof, the heavy chain variable region of the antibody has an amino acid sequence set forth in SEQ ID NO: 1, and the light chain variable region of the antibody has an amino acid sequence set forth in SEQ ID NO: 6;
the heavy chain variable region of the antibody has an amino acid sequence set forth in SEQ ID NO: 11, and the light chain variable region of the antibody has an amino acid sequence set forth in SEQ ID NO: 19;
the heavy chain variable region of the antibody has an amino acid sequence set forth in SEQ ID NO: 17, and the light chain variable region of the antibody has an amino acid sequence set forth in SEQ ID NO: 19;
the heavy chain variable region of the antibody has an amino acid sequence set forth in SEQ ID NO: 13, and the light chain variable region of the antibody has an amino acid sequence set forth in SEQ ID NO: 21;
the heavy chain variable region of the antibody has an amino acid sequence set forth in SEQ ID NO: 13, and the light chain variable region of the antibody has an amino acid sequence set forth in SEQ ID NO: 23;
the heavy chain variable region of the antibody has an amino acid sequence set forth in SEQ ID NO: 15, and the light chain variable region of the antibody has an amino acid sequence set forth in SEQ ID NO: 21;
the heavy chain variable region of the antibody has an amino acid sequence set forth in SEQ ID NO: 15, and the light chain variable region of the antibody has an amino acid sequence set forth in SEQ ID NO: 23;
the heavy chain variable region of the antibody has an amino acid sequence set forth in SEQ ID NO: 11, and the light chain variable region of the antibody has an amino acid sequence set forth in SEQ ID NO: 25; or
the heavy chain variable region of the antibody has an amino acid sequence set forth in SEQ ID NO: 17, and the light chain variable region of the antibody has an amino acid sequence set forth in SEQ ID NO: 25;
preferably, for the anti-TIGIT antibody or the antigen-binding fragment thereof, the antibody comprises a non-CDR region derived from a species other than murine, for example, from a human antibody;
preferably, for the anti-TIGIT antibody or the antigen-binding fragment thereof, the antibody comprises a heavy chain constant region that is an Ig gamma-1 chain C region (e.g., NCBI ACCESSION: P01857), and a light chain constant region that is an Ig kappa chain C region (e.g., NCBI ACCESSION: P01834);
preferably, for the anti-TIGIT antibody or the antigen-binding fragment thereof, the anti-TIGIT antibody or the antigen-binding fragment thereof is selected from Fab, Fab', F(ab')₂, Fd, Fv, dAb, a complementarity determining region fragment, a single chain antibody, a humanized antibody, a chimeric antibody, or a diabody;
preferably, for the anti-TIGIT antibody or the antigen-binding fragment thereof, the antibody binds to TIGIT-mFc with a K_{D} less than 4E-10 or less than 4E-11; preferably, the K_{D} is measured by a Fortebio molecular interaction instrument;
preferably, for the anti-TIGIT antibody or the antigen-binding fragment thereof, the antibody binds to TIGIT-mFc with an EC₅₀ less than 1.5 nM, less than 1.2 nM, or less than 1 nM; preferably, the EC₅₀ is measured by a flow cytometer;
preferably, the anti-TIGIT antibody is a monoclonal antibody, a humanized antibody, a chimeric antibody, or a multispecific antibody (e.g., a bispecific antibody);
preferably, the antigen-binding fragment is selected from Fab, Fab', F(ab')₂, Fd, Fv, dAb, Fab/c, a complementarity determining region fragment, a single chain antibody (e.g., scFv), a humanized antibody, a chimeric antibody, or a bispecific antibody;
preferably, for the anti-TIGIT antibody or the antigen-binding fragment thereof, the antibody is an antibody produced by hybridoma cell line LT019 deposited at China Center for Type Culture Collection (CCTCC) with an accession number of CCTCC NO: C2020208.

3. The pharmaceutical composition or the kit according to claim 1, for the anti-CTLA4-anti-PD-1 bispecific antibody, wherein
an amino acid sequence of the heavy chain variable region of the immunoglobulin is selected from SEQ ID NO: 27 and SEQ ID NO: 43, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; an amino acid sequence of the light chain variable region of the immunoglobulin is selected from SEQ ID NO: 28 and SEQ ID NO: 44, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; an amino acid sequence of the heavy chain variable region of the single chain antibody is selected from SEQ ID NO: 35, SEQ ID NO: 45, SEQ ID NO: 46, SEQ ID NO: 47, and SEQ ID NO: 48, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; an amino acid sequence of the light chain variable region of the single chain antibody is selected from SEQ ID NO: 36, SEQ ID NO: 49, SEQ ID NO: 50, SEQ ID NO: 51, and SEQ ID NO: 52, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
or
an amino acid sequence of the heavy chain variable region of the immunoglobulin is selected from SEQ ID NO: 35, SEQ ID NO: 45, SEQ ID NO: 46, SEQ ID NO: 47, and SEQ ID NO: 48, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; an amino acid sequence of the light chain variable region of the immunoglobulin is selected from SEQ ID NO: 36, SEQ ID NO: 49, SEQ ID NO: 50, SEQ ID NO: 51, and SEQ ID NO: 52, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; an amino acid sequence of the heavy chain variable region of the single chain antibody is selected from SEQ ID NO: 27 and SEQ ID NO: 43, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; an amino acid sequence of the light chain variable region of the single chain antibody is selected from SEQ ID NO: 28 and SEQ ID NO: 44, or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
preferably, for the anti-CTLA4-anti-PD-1 bispecific antibody, the bispecific antibody is selected from any one of the following (1) - (20):
(1) the heavy chain variable region of the immunoglobulin has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 35 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 36 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(2) the heavy chain variable region of the immunoglobulin has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 45 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 49 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(3) the heavy chain variable region of the immunoglobulin has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 46 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 50 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(4) the heavy chain variable region of the immunoglobulin has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 35 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 36 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(5) the heavy chain variable region of the immunoglobulin has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 45 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 49 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(6) the heavy chain variable region of the immunoglobulin has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 46 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 50 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(7) the heavy chain variable region of the immunoglobulin has an amino acid sequence set forth in SEQ ID NO: 35 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 36 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(8) the heavy chain variable region of the immunoglobulin has an amino acid sequence set forth in SEQ ID NO: 35 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 36 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(9) the heavy chain variable region of the immunoglobulin has an amino acid sequence set forth in SEQ ID NO: 45 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 49 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(10) the heavy chain variable region of the immunoglobulin has an amino acid sequence set forth in SEQ ID NO: 45 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 49 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(11) the heavy chain variable region of the immunoglobulin has an amino acid sequence set forth in SEQ ID NO: 46 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 50 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(12) the heavy chain variable region of the immunoglobulin has an amino acid sequence set forth in SEQ ID NO: 46 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 50 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(13) the heavy chain variable region of the immunoglobulin has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 47 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 51 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(14) the heavy chain variable region of the immunoglobulin has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 48 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 52 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(15) the heavy chain variable region of the immunoglobulin has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 47 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 51 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(16) the heavy chain variable region of the immunoglobulin has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 48 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 52 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(17) the heavy chain variable region of the immunoglobulin has an amino acid sequence set forth in SEQ ID NO: 47 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 51 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(18) the heavy chain variable region of the immunoglobulin has an amino acid sequence set forth in SEQ ID NO: 48 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 52 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 27 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 28 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
(19) the heavy chain variable region of the immunoglobulin has an amino acid sequence set forth in SEQ ID NO: 47 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 51 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
and
(20) the heavy chain variable region of the immunoglobulin has an amino acid sequence set forth in SEQ ID NO: 48 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the immunoglobulin has an amino acid sequence set forth in SEQ ID NO: 52 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto; the heavy chain variable region of the single chain antibody has an amino acid sequence set forth in SEQ ID NO: 43 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 44 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
preferably, for the anti-CTLA4-anti-PD-1 bispecific antibody,
the heavy chain of the immunoglobulin has an amino acid sequence set forth in SEQ ID NO: 53 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain thereof has an amino acid sequence set forth in SEQ ID NO: 54 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
preferably, for the anti-CTLA4-anti-PD-1 bispecific antibody, the first protein functional region is linked to the second protein functional region either directly or via a linker fragment, and/or the heavy chain variable region of the single chain antibody is linked to the light chain variable region of the single chain antibody either directly or via a linker fragment;
preferably, for the anti-CTLA4-anti-PD-1 bispecific antibody, the linker fragment is (GGGGS)n, n being a positive integer; preferably, n is 1, 2, 3, 4, 5, or 6;
preferably, for the anti-CTLA4-anti-PD-1 bispecific antibody, the numbers of the first protein functional region and the second protein functional region are each independently 1, 2, or more;
preferably, for the anti-CTLA4-anti-PD-1 bispecific antibody, the single chain antibody (preferably the heavy chain variable region) is linked to the C terminus of the heavy chain of the immunoglobulin;
preferably, for the anti-CTLA4-anti-PD-1 bispecific antibody,
the immunoglobulin is of human IgG1 subtype,
wherein, according to the EU numbering system, the heavy chain constant region of the immunoglobulin has one of the combinations of the following mutations:
L234A and L235A; or
L234A and G237A; or
L235A and G237A; or
L234A, L235A, and G237A;
preferably, for the anti-CTLA4-anti-PD-1 bispecific antibody, the bispecific antibody comprises:
a first protein functional region targeting PD-1 and
a second protein functional region targeting CTLA4,
the number of the first protein functional region is 1, and the number of the second protein functional region is 2;
wherein the first protein functional region is an immunoglobulin, and the second protein functional region is a single chain antibody;
the heavy chain of the immunoglobulin has an amino acid sequence set forth in SEQ ID NO: 53 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain thereof has an amino acid sequence set forth in SEQ ID NO: 54 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
the heavy chain variable region of the single chain antibody has an amino acid sequence set forth in SEQ ID NO: 48 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto, and the light chain variable region of the single chain antibody has an amino acid sequence set forth in SEQ ID NO: 52 or a sequence having at least 60%, at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, 92%, 93%, 94%, at least 95%, at least 96%, at least 97%, at least 98% or at least 99% homology thereto;
the single chain antibody is linked to the C terminus of the heavy chain of the immunoglobulin;
the first protein functional region is linked to the second protein functional region via a first linker fragment; the heavy chain variable region of the single chain antibody is linked to the light chain variable region of the single chain antibody via a second linker fragment; the first linker fragment and the second linker fragment are identical or different;
preferably, the first linker fragment and the second linker fragment each have an amino acid sequence independently selected from SEQ ID NO: 55 and SEQ ID NO: 56;
preferably, amino acid sequences of the first linker fragment and the second linker fragment are set forth in SEQ ID NO: 56;
preferably, the heavy chain of the anti-CTLA4-anti-PD-1 bispecific antibody has an amino acid sequence set forth in SEQ ID NO: 57, the light chain has an amino acid sequence set forth in SEQ ID NO: 59, and the bispecific antibody has a structure of IgG-scFv, wherein the IgG part is an anti-PD1 antibody, and the scFv part is an anti-CTLA4 antibody,
wherein the HCDR1 sequence of the anti-PD1 antibody is set forth in SEQ ID NO: 61, the HCDR2 sequence is set forth in SEQ ID NO: 62, the HCDR3 sequence is set forth in SEQ ID NO: 63, the VH sequence is set forth in SEQ ID NO: 73, the LCDR1 sequence of the anti-PD1 antibody is set forth in SEQ ID NO: 70, the LCDR2 sequence is set forth in SEQ ID NO: 71, the LCDR3 sequence is set forth in SEQ ID NO: 72, and the VL sequence is set forth in SEQ ID NO: 76;
the HCDR1 sequence of the anti-CTLA4 antibody is set forth in SEQ ID NO: 64, the HCDR2 sequence is set forth in SEQ ID NO: 65, the HCDR3 sequence is set forth in SEQ ID NO: 66, the VH sequence is set forth in SEQ ID NO: 74, the LCDR1 sequence of the anti-CTLA4 antibody is set forth in SEQ ID NO: 67, the LCDR2 sequence is set forth in SEQ ID NO: 68, the LCDR3 sequence is set forth in SEQ ID NO: 69, and the VL sequence is set forth in SEQ ID NO: 75.

4. The pharmaceutical composition according to any of claims 1-3, wherein the anti-TIGIT antibody or the antigen-binding fragment thereof and the anti-CTLA4-anti-PD-1 antibody or the antigen-binding fragment thereof are present in a mass ratio, on antibody basis, of 1:5-5: 1, for example, 1:5, 1:4, 1:3, 1:2, 1:1, 2:1, 3:1, 4:1, or 5:1.

5. A kit comprising a first product and a second product in separate packages, wherein
the first product comprises the anti-TIGIT antibody or the antigen-binding fragment thereof as defined in any of claims 1-4;
the second product comprises the anti-CTLA4-anti-PD-1 bispecific antibody or the antigen-binding fragment thereof as defined in any of claims 1-4;
preferably, the kit further comprises a third product in a separate package comprising one or more chemotherapeutic drugs,
preferably, the first product and the second product further independently comprise one or more pharmaceutically acceptable adjuvants;
preferably, the combination product further comprises a package insert;
preferably, for the kit, the anti-TIGIT antibody or the antigen-binding fragment thereof and the anti-CTLA4-anti-PD-1 bispecific antibody or the antigen-binding fragment thereof are present in a mass ratio, on antibody basis, of 1:5-5:1, for example, 1:5, 1:4, 1:3, 1:2, 1:1, 2:1, 3:1, 4:1, or 5:1.

6. A method for treating and/or preventing a tumor, comprising: administering to a subject in need an effective amount of the anti-TIGIT antibody or the antigen-binding fragment thereof as defined in any of claims 1-4 and/or the anti-CTLA4-anti-PD-1 bispecific antibody or the antigen-binding fragment thereof as defined in any of claims 1-4;
wherein preferably, one or more drugs are further administered in combination (e.g., a chemotherapeutic agent or a growth inhibitor, a targeted therapeutic agent, an antibody-drug conjugate, an antimetabolite, an antibiotic, an anti-hormonal agent, a plant-based anticancer agent, and/or a hormonal drug), wherein preferably, the drug is selected from one or more of the following drugs: adriamycin, tamoxifen, megestrol, asparaginase, a platinum-based drug (e.g., cisplatin, carboplatin, or oxaliplatin), a fluorouracil antineoplastic drug, cyclophosphamide, pemetrexed, paclitaxel, vinca alkaloids, adriamycin, goserelin, an alkylating agent, an anthracycline, an anti-androgen agent, an aromatase inhibitor, a protein kinase inhibitor (e.g., a tyrosine kinase inhibitor), a lipid kinase inhibitor, an antisense oligonucleotide, a ribozyme, a topoisomerase inhibitor, a cytotoxic agent, an anti-tumor antibiotic, a proteasome inhibitor, an anti-microtubule agent, an EGFR antagonist, a retinoid, a histone deacetylase inhibitor, a B-raf inhibitor, an MEK inhibitor, a K-ras inhibitor, a c-Met inhibitor, an Alk inhibitor, a phosphatidylin-ositol-3-kinase inhibitor, an Akt inhibitor, an mTOR inhibitor, a dual phosphatidylinositol-3-kinase and mTOR inhibitor, maytansine, monomethyl auristatin E, calicheamicin, esperamicin, and a radioisotope chelating agent;
preferably, the anti-TIGIT antibody, the anti-CTLA4-anti-PD-1 bispecific antibody, and the anti-tumor chemotherapeutic drug are administered simultaneously or sequentially; more preferably, the anti-TIGIT antibody and the anti-CTLA4-anti-PD-1 bispecific antibody are administered before or after a surgical treatment, and/or before or after a radiation therapy;
preferably, the anti-TIGIT antibody, the anti-CTLA4-anti-PD-1 bispecific antibody and/or the chemotherapeutic drug are in a form suitable for intravenous injection or intravenous drip infusion, preferably in a liquid form;
preferably, the tumor is selected from one or more of the following:
breast cancer, ovarian cancer, colorectal cancer, cervical tumor, multiple myeloma, non-Hodgkin's lymphoma, B-lymphoma, plasma cell cancer, head and neck cancer, brain cancer, throat cancer, nasopharyngeal cancer, oesophageal cancer, esophageal squamous cancer, thyroid cancer, mesothelioma, adenocarcinoma (e.g., pancreatic cancer), lung cancer (e.g., non-small cell lung cancer and small cell lung cancer), breast cancer, liver cancer (e.g., hepatocellular carcinoma and hepatobiliary cancer), gastric cancer, gastrointestinal cancer, intestinal cancer (e.g., colon cancer and colorectal cancer), biliary tract cancer (e.g., cholangiocarcinoma), kidney cancer, fallopian tube cancer, endometrial cancer, cervical cancer, bladder cancer, urothelial carcinoma, prostate cancer, testicular cancer, skin cancer, melanoma, myeloma (e.g., multiple myeloma), non-Hodgkin's lymphoma, B-lymphoma, plasma cell carcinoma, leukemia, lymphoma, bone cancer, osteosarcoma, chondrosarcoma, high microsatellite instability (MSI-H) or mismatch repair deficient (dMMR) solid tumors;
preferably, the unit dose of the anti-TIGIT antibody and/or the anti-CTLA4-anti-PD-1 bispecific antibody as defined in any of claims 1-4 is 0.1-100 mg, preferably 1-10 mg, per kg body weight; alternatively, the unit dose of the anti-TIGIT antibody according to any of claims 1-4 and/or the anti-CTLA4-anti-PD-1 bispecific antibody according to any of claims 1-4 is 10-1000 mg, preferably 50-500 mg, 100-400 mg, 150-300 mg, 150-250 mg, or 200 mg, in each subject;
preferably, the dose is administered from twice daily to about once every other day, or once every 3 days, 4 days, 5 days, 6 days, 10 days, 1 week, 2 weeks, 3 weeks, 4 weeks, 5 weeks, or 6 weeks;
preferably, the route of administration is intravenous drip infusion or intravenous injection.

7. A unit formulation, preferably used for treating a tumor, comprising: 1-10000 mg (preferably 10-1000 mg, preferably 50-500 mg, 100-400 mg, 150-300 mg, 150-250 mg, or 200 mg) of the anti-TIGIT antibody as defined in any of claims 1-4, 1-10000 mg (preferably 1-1000 mg, preferably 50-500 mg, 100-400 mg, 150-300 mg, 150-250 mg, 200 mg or 100 mg) of the anti-CTLA4-anti-PD-1 bispecific antibody as defined in any of claims 1-4, and optionally one or more of the chemotherapeutic drugs (such as a platinum-based drug and/or a fluorouracil antineoplastic drug) as defined in claim 6, wherein the anti-TIGIT antibody, the anti-CTLA4-anti-PD-1 bispecific antibody and the chemotherapeutic drug are packaged separately.

8. A single dose unit, preferably used for treating a tumor, comprising: 0.1-10000 mg (preferably 1-1000 mg, preferably 50-500 mg, 100-400 mg, 150-300 mg, 150-250 mg, 200 mg or 100 mg) of the anti-TIGIT antibody as defined in any of claims 1-4 and 0.1-10000 mg (preferably 1-1000 mg, preferably 50-500 mg, 100-400 mg, 150-300 mg, 150-250 mg, 200 mg or 100 mg) of the anti-CTLA4-anti-PD-1 bispecific antibody as defined in any of claims 1-4.
